# EUROPEAN PATENT APPLICATION

(11) **EP 4 144 416 A1**
(43) Date of publication of application: **08.03.2023**
(21) Application number: 22151994.5
(22) Date of filing: 07.09.2016
(51) Int. Cl.: A61Q 17/04, A61K 8/73, A61K 8/04

(54) **FOAMING SUNSCREEN COMPOSITION CONTAINING AN ULTRAVIOLET RADIATION-ABSORBING COMPOUND AND A SUPERHYDROPHILIC AMPHIPHILIC COPOLYMER**

(30) Priority: 18.09.2015 US 201514858164
(62) Divisional of application: 16766772.4
(71) Applicant: Johnson & Johnson Consumer Inc., Skillman, NJ 08558 (US)
(72) Inventor: DALY,, Susan, Skillman, 08558 (US); MAITRA,, Prithwiraj, Skillman, 08558 (US); SETIAWAN,, Barry, Skillman, 08558 (US)
(74) Representative: Carpmaels & Ransford LLP

(57) **Abstract**

Foaming sunscreen compositions of the present invention include a continuous water phase containing from about 0.75% to about 6% of a superhydrophilic amphiphilic copolymer and a discontinuous oil phase dispersed in the water phase, which oil phase contains a UV-absorbing compound, and which foaming sunscreen compositions are essentially free of monomeric surfactants and substantially free of oil soluble polymers.

## Description

### FIELD OF THE INVENTION

The present invention relates to topically-acceptable, foaming sunscreen compositions in the form of oil-in-water emulsions comprising a UV-absorbing compound and a superhydrophilic amphiphilic copolymer, which compositions exhibit low irritation and high flash-foaming.

### BACKGROUND OF THE INVENTION

The prolonged exposure to UV radiation, such as from the sun, can lead to the formation of light dermatoses and erythemas, as well as increase the risk of skin cancers, such as melanoma, and accelerate skin aging, such as loss of skin elasticity and wrinkling.

Numerous sunscreen compositions are commercially available with varying ability to shield the body from ultraviolet light. Unfortunately, many commercial foamable sunscreens either sting the eye or irritate the skin. Accordingly, sunscreen formulations that are both mild and capable of forming a high volume of "flash foam" with relatively low energy input are desired by the consumer.

The challenge of creating mild and foamable sunscreens is further magnified if one imposes additional constraints on the sunscreen composition. For example, the inventors have recognized that it would be desirable to have mild, foamable sunscreen compositions that include a polymeric surfactant compound (e.g., a superhydrophilic amphiphilic copolymer) and are essentially free of monomeric surfactant.

### SUMMARY OF THE INVENTION

Foaming sunscreen compositions of the present invention include an oil-in-water emulsion comprising a continuous water phase containing from about 0.75% to about 6% by weight of a superhydrophilic amphiphilic copolymer, based on total weight of the composition, and a discontinuous oil phase dispersed in the water phase, which oil phase contains a UV-absorbing compound. The compositions are essentially free of, or free of, monomeric surfactants, and substantially free of oil soluble polymers. The invention also includes a non-aerosol mechanical dispensing container comprising the foaming sunscreen composition contained therein, which container and composition are free of a propellant gas.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. As used herein, unless otherwise indicated, all alkyl, alkenyl, and alkoxy groups may be straight or branched chain groups. As used herein, unless otherwise indicated, the term "molecular weight" refers to weight average molecular weight, (Mw).

Unless defined otherwise, all concentrations refer to concentrations by weight. Also, unless defined otherwise, the term "essentially free of," with respect to a class of ingredients, refers to the particular ingredient(s) being present in a concentration less than is necessary for the particularly ingredient to be effective to provide the benefit or property for which it otherwise would be used, for example, 0.75% or less by weight, such as about 0.5% or less by weight.

As used herein, "foaming composition" means a composition that is capable of forming a foam in the absence of a propellant gas when dispensed from a non-aerosol mechanical dispensing container. Such containers may comprise a dispensing head with a housing enclosing a pump mechanism and a screen material in the flow path to convert the foaming composition into a foam, and a dip-tube for delivering the foaming composition from the container to the dispensing head. Suitable dispensing containers are described in U. S. Patent No. 6,660,282, the content of which is hereby incorporated by reference in its entirety. Suitable foaming apparatuses are commercially available, e.g. as pump foam systems, from the companies Airspray (the Netherlands), Keltec (the Netherlands), Ophardt (Germany), Brightwell (UK), Taplast (Italy) and Supermatic (Switzerland). Suitable foaming apparatuses include, e.g. Taplastfoamer Pump PIUMA 262/400, Taplast foamer pump PIUMA 263/400, Rexam Airspray: F2-Elegant Finger Pump Foamer, Rexam Airspray: F3-Elegant Finger Pump Foamer, Rexam Airspray: G3-Elegant Finger Pump Foamer, Rexam Airspray: M3-Mini Foamer, Rexam Airspray: T1-Table Top Foamer and, from Rieke Packaging Systems, the models RF-08 Finger Tip Foamer and RF-17 Palm Foamer.

### SUPERHYDROPHILIC AMPHIPHILIC COPOLYMER

As used herein, the term "superhydrophilic amphiphilic copolymer," ("SAC") is defined as a copolymer that may be represented by the following general structure: 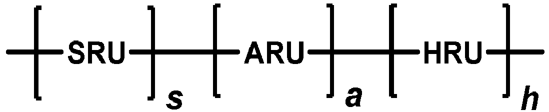 wherein an "SRU" is a superhydrophilic repeat unit as defined herein, an "ARU" is an amphiphilic repeat unit as defined herein, an "HRU" is a hydrophilic repeat unit as defined herein, wherein *s*≥ 2, *a* >0, *h*≥ 0, and the total number of repeat units, *s*+*a*+*h* is between 4 and about 1000. The term "between," when used herein to specify a range such as "between 4 and about 1000," is inclusive of the endpoints, e.g. "4" and "about 1000." The total number of repeat units in the SAC is based on the weight-average molecular weight (Mw) of the SAC; thus the number of repeat units, as discussed herein are "weight average" as well. Further, all molecular weights described herein are in the units of Daltons (Da). As will be recognized by one of skill in the art, the pattern of repeat units (SRUs, ARUs, HRUs) incorporated in SACs of the present invention are generally random; however, they may also have alternating, statistical, or blocky incorporation patterns. In addition, SAC architectures may be linear, star-shaped, branched, hyperbranched, dendritic, or the like.

Those of skill in the art will recognize that total number of repeat units in a SAC (SRUs + ARUs + HRUs, i.e. s + a + h in the above formula) is synonymous with the term "degree of polymerization" ("DP") of the SAC.

A "repeat unit" as defined herein and known the art is the smallest atom or group of atoms (with pendant atoms or groups, if any) comprising a part of the essential structure of a macromolecule, oligomer, block, or chain, the repetition of which constitutes a regular macromolecule, a regular oligomer molecule, a regular block, or a regular chain (definition from Glossary of Basic Terms in Polymer Science, A. D. Jenkins et al. Pure Appl. Chem. 1996 68, 2287-2311.)

As will be recognized by those of skill in the art in light of the description herein and knowledge of the art, the backbone of a polymer derived from ethylenically-unsaturated monomers comprises repeat units including one or two, or in the case of alternating polymers four, carbon atoms that were unsaturated in the monomers prior to polymerization, and any pendant groups of such carbons. For example, polymerization of an ethylenically-unsaturated monomer of the formula: (A)(Y)C=C(B)(Z) will generally result in a polymer comprising repeat units of the formula: 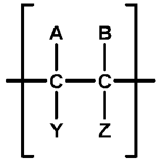 comprising the two previously unsaturated carbons of the monomer and their pendant groups (examples of which are described herein below, for example in the descriptions of SRUs, ARUs, and HRUs). However, if the pendant groups of the two carbons are the same such that, for example in the formula above, A-C-Y and B-C-Z are the same moiety, then each of such one carbon units and its pendant groups (A-C-Y or B-C-Z, being the same) are considered to be the repeat unit comprising only one previously unsaturated carbon from the monomer (e.g. the repeat unit of a homopolyer derived from ethylene, H₂C=CH₂ is [-[CH₂]-] not [-[CH₂CH₂]-]. With regard only to alternating copolymers which, as known in the art, are defined as those polymers in which the repeat units derived from the two comonomers alternate consistently throughout the polymer (as opposed to the random polymerization of co-monomers to form a polymer in which repeat units derived from the two monomers are randomly linked throughout the polymer or the block copolymerization of comonomers to form non-alternating blocks of repeat units derived from the two monomers), the repeat unit is defined as the unit derived from one of each of the co-monomers comprising four carbons that were previously ethylenically-unstaurated in the two comonomers prior to polymerization. That is, maleic anhydride and vinyl methyl ether are used in the art to form an alternating copolymer, poly(maleic anhydride-*alt*-vinyl methyl ether) having repeat units of the structure:

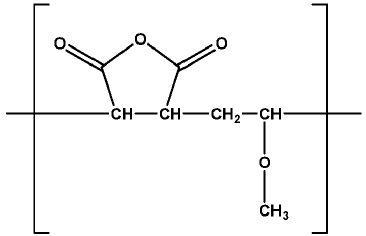

For saccharide-based polymers whose backbone is formed by linking sugar rings, the repeat unit generally comprises the sugar ring and pendant groups (as shown herein below, for example in the descriptions of SRUs, ARUs, and HRUs). Examples of such repeat units also include sugar ring repeat units with pendant sugar rings, for example, Glactomannans are polysaccharides comprised of a mannose (monosaccharide-based) backbone. Pending from some but not all of the mannose groups in the backbone (and arranged in either a random or block fashion) are pendant galactose groups. As will be readily understood by one skilled in the art, this structure is best described as having, two repeat units, mannose and mannose-galactose.

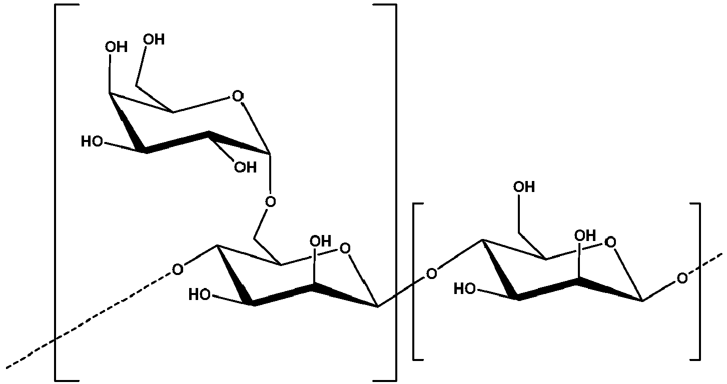

For alternating saccharide-based polymers, then the repeat unit is the two sugar rings derived from the alternating sugar-based monomers and their pendant groups. For example, Hyaluronan is an alternating saccharide copolymer derived from two saccharides, D-glucuronic acid and D-N-acetylglucosamine that alternate to give a disaccharide repeat units.

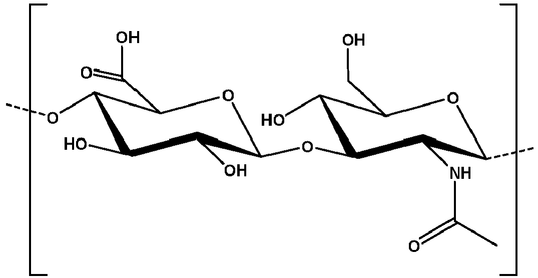

A "hydrophobic moiety" is hereby defined as a nonpolar moiety that contains at least one of the following: (a) a carbon-carbon chain of at least four carbons in which none of the four carbons is a carbonyl carbon or has a hydrophilic moiety bonded directly to it; (b) two or more alkyl siloxy groups (-[Si(R)₂-O]-); and/or (c) two or more oxypropylene groups in sequence. A hydrophobic moiety may be, or include, linear, cyclic, aromatic, saturated or unsaturated groups. In certain embodiments, hydrophobic moieties comprise a carbon chain of at least six or more carbons, more preferably seven or more carbons in which none of the carbons in such chain have a hydrophilic moiety bonded directly thereto. Certain other hydrophobic moieties include moieties comprising a carbon chain of about eight or more carbon atoms, more preferably about 10 or more carbon atoms in which none of the carbons in such chain have a hydrophilic moiety bonded directly thereto. Examples of hydrophobic functional moieties may include esters, ketones, amides, carbonates, urethanes, carbamates, or xanthate functionalities, and the like, having incorporated therein or attached thereto a carbon chain of at least four carbons in which none of the four carbons has a hydrophilic moiety bonded directly to it. Other examples of hydrophobic moieties include groups such as poly(oxypropylene), poly(oxybutylene), poly(dimethylsiloxane), fluorinated hydrocarbon groups containing a carbon chain of at least four carbons in which none of the four carbons has a hydrophilic moiety bonded directly to it, and the like.

As used herein, the term "hydrophilic moiety," is any anionic, cationic, zwitterionic, or nonionic group that is polar. Nonlimiting examples include anionics such as sulfate, sulfonate, carboxylic acid/carboxylate, phosphate, phosphonates, and the like; cationics such as: amino, ammonium, including mono-, di-, and trialkylammoniumspecies, pyridinium, imidazolinium, amidinium, poly(ethyleneiminium), and the like; zwitterionics such as ammonioalkylsulfonate, ammonioalkylcarboxylate, amphoacetate, and the like; and nonionics such as hydroxyl, sulfonyl, ethyleneoxy, amido, ureido, amine oxide, and the like.

As used herein, the term "superhydrophilic repeat unit," ("SRU") is defined as a repeat unit that comprises two or more hydrophilic moieties and no hydrophobic moieties. For example, SRUs may be derived from ethylenically-unsaturated monomers having two or more hydrophilic moieties and no hydrophobic moieties, including repeat units of the general formulae: 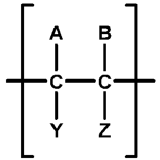 wherein A, B, Y, and Z collectively include at least two hydrophilic moieties and no hydrophobic moieties; or 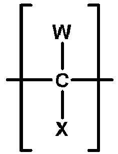 wherein W and X collectively include at least two hydrophilic moieties. Illustrative examples of such SRUs include, but are not limited to, those derived from superhydrophilic monomers described herein and the like, such as: 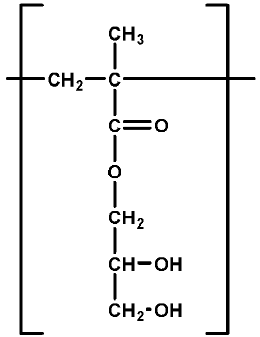 which is derived from glyceryl methacrylate; or others such as 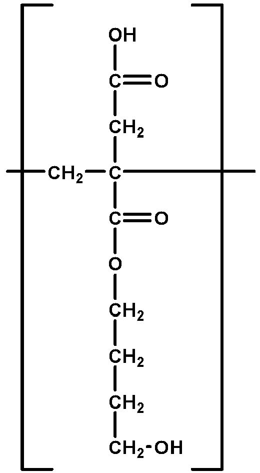 which is derived from 4-Hydroxybutyl itaconate; and the like.

Other examples of SRUs include saccharide-based repeat units including repeat units derived from fructose, glucose, galactose, mannose, glucosamine, mannuronic acid, guluronic acid, and the like, such as: 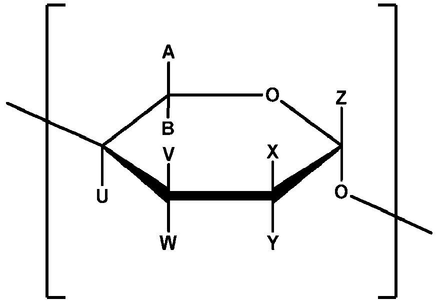 wherein A, B, U, V, W, X, Y, and Z collectively include at least two hydrophilic moieties and no hydrophobic moieties, one example of which includes 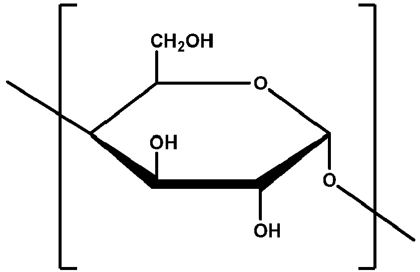 which is a α(1→4)-D-glucose SRU; or 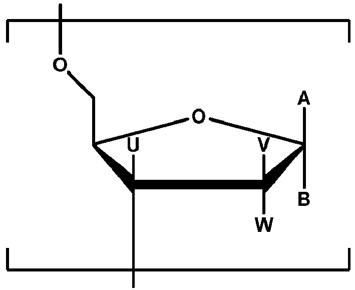 wherein A, B, U, V, and W collectively include at least two hydrophilic moieties and no hydrophobic moieties, one example of which includes 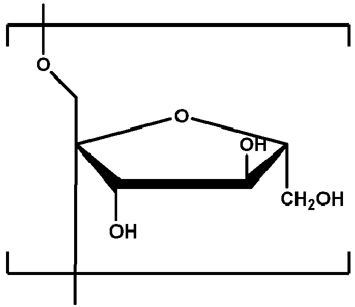 a β(2→1)-D-fructose SRU; and the like. As will be recognized by those of skill in the art, monosaccharide repeat units may be linked in various fashions, that is, through various carbons on the sugar ring e.g. (1→4), (1→6), (2→1), etc. Any of such linkages, or combinations thereof, may be suitable for use herein in monosaccharide SRUs, ARUs, or HRUs.

Other examples of SRUs include repeat units derived from amino acids, including, for example, repeat units of the formula: 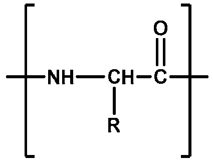 wherein R includes a hydrophilic repeat unit, examples of which include an aspartic acid SRU, and the like.

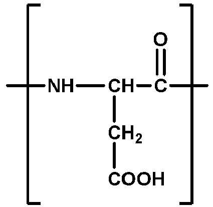

As used herein, the term "amphiphilic repeat unit," ("ARU") is defined as a repeat unit that comprises at least one hydrophilic moiety and at least one hydrophobic moiety. For example, ARUs may be derived from ethylenically-unsaturated monomers having at least one hydrophilic moiety and at least one hydrophobic moiety, including repeat units of the general formulae 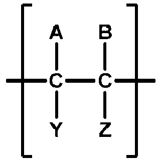 wherein A, B, Y, and Z collectively include at one hydrophilic moiety and at least one hydrophobic moiety; or 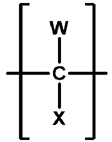 wherein W and X collectively include at one hydrophilic moiety and at least one hydrophobic moiety; examples of which include 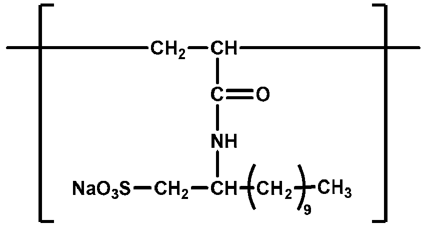 sodium 2-acrylamidododecylsulfonate amphiphilic repeat unit (ARU), and the like.

Other examples of ARUs include saccharide-based repeat units including repeat units derived from including repeat units derived from fructose, glucose, galactose, mannose, glucosamine, mannuronic acid, guluronic acid, and the like, such as: 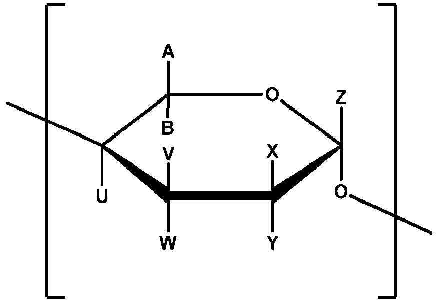 wherein A, B, U, V, W, X, Y, and Z collectively include at least one hydrophilic moiety and at least one hydrophobic moiety, or 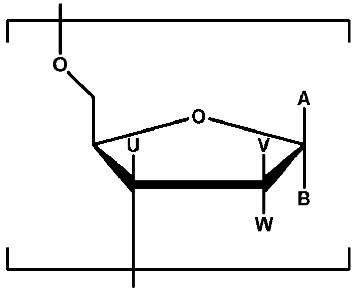 wherein A, B, U, V, and W collectively include at least one hydrophilic moiety and at least one hydrophobic moiety, examples of which include 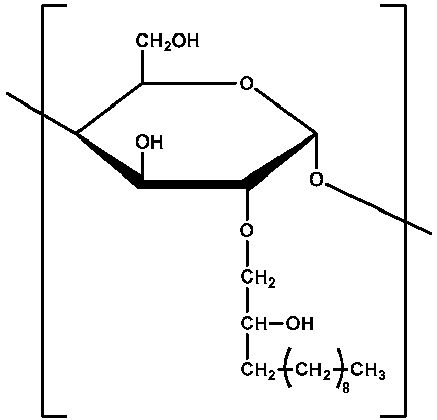 1,2-epoxydodecane modified α (1→4)-D-glucose ARU, and the like.

Other examples of ARUs include repeat units derived from amino acids, including, for example, repeat units of the formula: 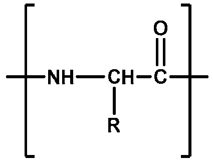 wherein R includes a hydrophobic group, examples of which include 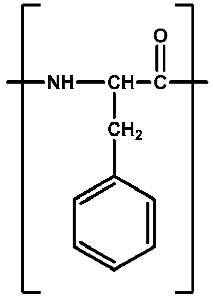 a phenylalanine ARU; and the like.

As will be readily understood by those of skill in the art, the term "hydrophilic repeat unit," ("HRU") is defined as a repeat unit that comprises one and only one hydrophilic moiety and no hydrophobic moieties. For example, HRUs may be derived from ethylenically-unsaturated monomers having one and only one hydrophilic moiety and no hydrophobic moieties, including repeat units of the general formulae 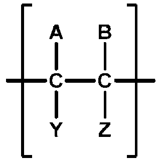 wherein A, B, Y, and Z collectively include one and only one hydrophilic moiety and no hydrophobic moieties; or 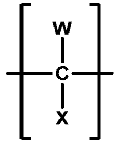 wherein W and X collectively include one and only one hydrophilic moiety and no hydrophobic moieties, examples of which include 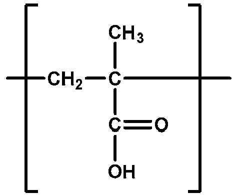 methacrylic acid hydrophilic repeat unit (HRU); and the like.

Other examples of HRUs include saccharide-based repeat units including repeat units derived from fructose, glucose, galactose, mannose, glucosamine, mannuronic acid, guluronic acid, and the like, such as: 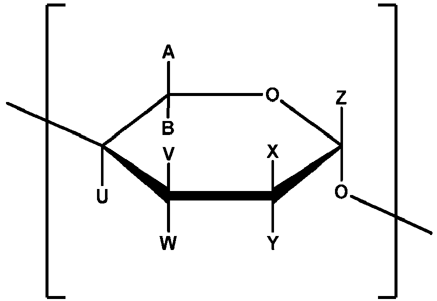 wherein A, B, U, V, W, X, Y, and Z collectively include one and only one hydrophilic moiety and no hydrophobic moieties, or 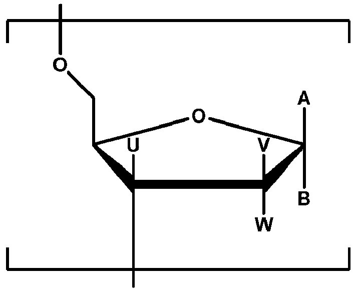 wherein A, B, U, V, and W collectively include one and only one hydrophilic moiety and no hydrophobic moieties. One example of saccharide-based hydrophilic repeat unit includes methylcellulose HRU, (methyl-substituted poly[β(1→4)-*D*-glucose], DS = 2.0)

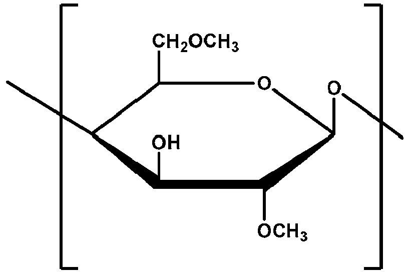

Other examples of HRUs include repeat units derived from amino acids, including, for example, repeat units of the formula: 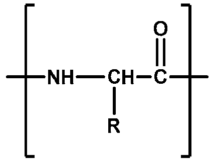 wherein R is neither a hydrophilic nor hydrophobic moiety, one example of which includes 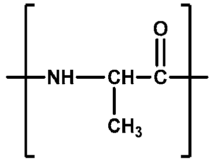 alanine HRU; and the like. As will be recognized by one of skill in the art, in any of the formulae herein, examples of moieties that are neither hydrophilic nor hydrophobic include hydrogen, C₁-C₃ alkyl, C₁-C₃ alkoxy, C₁-C₃ acetoxy, and the like.

According to certain embodiments of the invention, it has been discovered that SACs having a DP between 4 and about 1000 repeat units exhibit a significant and unexpected combination of low-irritation and high foaming properties. Examples of SACs suitable for use in such embodiments include those having a DP of between 4 and about 500, or 4 and about 200, or 4 and about 100, or 4 and about 50 repeat units. Other examples include those having a DP of between 5 and about 500, more preferably 5 and about 200, more preferably 5 and about 100, more preferably 5 and about 50 repeat units. Other examples include those having a DP of between 6 and about 200, more preferably 6 and about 100, more preferably 6 and about 50 repeat units. Other examples include those having a DP of between 7 and about 100, more preferably 7 and about 50 repeat units.

According to certain embodiments, it has further been discovered that certain SACs are capable of forming compositions having relatively low "Dynamic Surface Tension Reduction Time", (that is, the time required to reduce surface tension of pure water from 72 mN/m to 55 mN/m, "*t*_{*γ*=55}", associated with a particular composition, which value is measured conventionally via the Drop Shape Analysis Test ("DSA Test") described in further detail below) and are for use in compositions having significant and unexpected combinations of low-irritation and high foaming properties, as compared to comparable compositions. According to certain embodiments, the SACs of the present invention have a *t*_{*γ*=55} of about 120 seconds (s) or less. In certain other embodiments, the SACs of the present invention have a *t*_{*γ*=55} of about 75 seconds or less, or about 50 seconds or less, or about 45 seconds or less.

Drop Shape Analysis (DSA, also known as Pendant Drop Method or PDM) is a well-known method for measuring the static interfacial or surface tension as a function of time. The surface tension measured by DSA is determined by fitting the shape of the hanging drop (captured in a video image) to the Young-Laplace equation, which relates interfacial tension to drop shape. The Laplace equation is the mechanical equilibrium condition for two homogeneous fluids separated by an interface (Handbook of Applied Surface and Colloid Chemistry, Vol. 2; Holmberg, K., Ed.; John Wiley & Sons: Chicester, U.K., 2002, pp 222-223). It relates the pressure difference across a curved interface to the surface tension and the curvature of the interface: Solutions for the determination of surface tension may be prepared as follows: a polymer sample (1150 mg active solids) is diluted in Millipore-Q deionized water (200 mL) in an acid-washed glass flask with glass stopper. This stock solution is mixed by manually shaking for five minutes and allowed to stand overnight. A dilution (1/4) of the stock solution is prepared by further diluting the stock solution with Millipore-Q water in acid-washed glassware - this is the sample is used for DSA analysis. The samples are analyzed using a DSA 100 instrument (Krüss GmbH, Hamburg, Germany) operating at 25°C. The drop is monitored over 120 seconds and images are captured approximately every 0.16 seconds for the first 10 seconds, every 0.5 seconds for the next 50 seconds, and every second for the last 60 seconds. All of the captured images are analyzed to determine the surface tension at each time frame. Surface tension values are calculated using the Drop Shape Analysis (DSA) for Windows^{™} package (Krüss GmbH, Hamburg, Germany). Dynamic reduction of surface tension is reported as the time in seconds required to reduce the surface tension of the test solution to 55 mN/m, *t*_{*γ*=55}. The reported values of *t*_{*γ*=55} are the average of three individual measurement runs.

According to certain embodiments, SACs suitable for use in the present invention exhibit a mole percent (mol %) of amphiphilic repeat units (amphiphilic mol% = (a/s+a+h)) of less than 10%, such as those having a mol% of ARUs of from about 5 to about 10 mol%.

The SACs useful in the present invention may be of any suitable molecular weight, provided the required DP is met. In certain embodiments, the SAC has a weight average molecular weight from about 1000 grams/mol to about 200,000 grams/mol. In one embodiment, the SAC has a weight average molecular weight of from about 1000 to about 100,000, or from about 1,000 to about 75,000, or from about 1,000 to about 50,000, or from about 1,000 to about 25,000, or from about 1,000 to about 10,000, or from about 3,000 to about 10,000. Furthermore, according to certain embodiments, SACs useful in the present invention are provided in readily water-soluble, free flowing, solid forms, such as powders.

SACs suitable for use in the present invention include polymers of various chemical classifications, obtained via a variety of synthetic routes. Examples include polymers having a backbone that comprises, consists essentially of or consists of a plurality of carbon-carbon bonds, as well as polymers having a backbone comprising a plurality of carbon-heteroatom bonds. As will be recognized by those of skill in the art, the backbone refers generally to the portion of repeat units in a polymer that is covalently bonded to adjacent repeat units vs. "pendant groups".

Examples of synthetic routes for obtaining SACs of the present invention include copolymerization of (i) one or more ethylenically unsaturated amphiphilic comonomer(s) with (ii) one or more ethylenically unsaturated superhydrophilic comonomer(s), and optionally, (iii) one or more ethylenically unsaturated hydrophilic comonomer(s).

Nonlimiting examples of ethylenically unsaturated amphiphilic comonomers include those having the following structure: 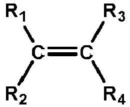
- where R₁ = R₂ = H, R₃ = H or CH₃, and R₄ comprises Amphiphilic (Amphil) group, or
- where R₁ = R₂ = H, R₃ comprises a hydrophilic group (Hphil), and R₄ comprises hydrophobic group (Hphob), or
- where R₁, R₃ are independently H or CH₃, R₂ comprises Hphil, and R₄ comprises Hphob group, or
- where R₁, R₄ are independently H or CH₃, R₃ comprises Hphil, and R₄ comprises Hphob group, or
- where R₂, R₃ are independently H or CH₃, R₁ comprises Hphil, and R₄ comprises Hphob group

### Anionic:

- ω-alkeneoates: e.g. sodium 11-undecenoate 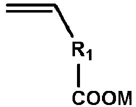 where R₁ = any linear or branched carbon chain containing more than 5 carbon atoms and M = H⁺, NH₄⁺, or any Group IA alkali metal cation.
- (Meth)acrylamidoalkylcarboxylates and (meth)acryloyloxyalkylcarboxylates: e.g. sodium 11-acrylamidoundecanoate, sodium 11-methacryloyloxyundecanoate 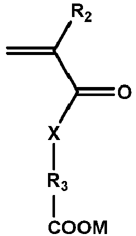 where R₂ = H or CH₃, X = O or NH, R₃ = any linear or branched carbon chain containing more than 5 carbon atoms and M = H⁺, NH₄⁺, or any Group IA alkali metal cation.
- (Meth)acrylamidoalkylsulfonic acids: e.g. 2-acrylamidododecylsulfonic acid 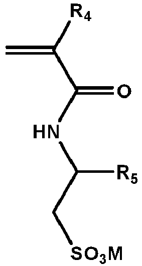 where R₄ = H or CH₃, X = O or NH, R₅ = any linear or branched carbon chain containing more than 5 carbon atoms and M = H⁺, NH₄⁺, or any Group IA alkali metal cation.
- Allylalkylsulfosuccinates: e.g. sodium allyldodecylsulfosuccinate (TREM LF-40, Cognis) 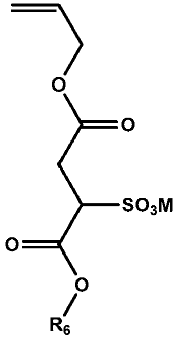 where R₆ = any linear or branched carbon chain containing more than 5 carbon atoms and M = H⁺, NH₄⁺, or any Group IA alkali metal cation.

### Cationic:

- Quaternized aminoalkyl(meth)acrylamides and aminoalkyl(meth)acrylates: e.g. (3-methacrylamidopropyl)dodecyldimethylammonium chloride, (2-methacryloyloxyethyl)dodecyl dimethylammonium chloride 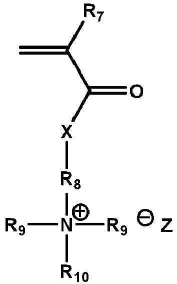 where R₇ = H or CH₃, X = O or NH, R₈ = any linear or branched carbon chain containing 5 or less carbon atoms, R₉ = H, CH₃, CH₂CH₃ or CH₂CH₂OH, R₁₀ = any linear or branched carbon chain containing more than 5 carbon atoms and Z = any Group VII-A halide anion, OR where R₇ = H or CH₃, X = O or NH, R₈ = any linear or branched carbon chain containing more than 5 carbon atoms, R₉, R₁₀ are independently H, CH₃, CH₂CH₃ or CH₂CH₂OH, and Z = any Group VII-A halide anion
- Quaternized vinylpyridines: e.g. (4-vinyl)dodecylpyridinium bromide 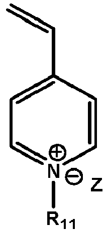 where R₁₁ = any linear or branched carbon chain containing more than 5 carbon atoms and Z = any Group VII-A halide anion.
- Alkyldiallylmethylammonium halides: e.g. diallyldodecylmethylammonium chloride 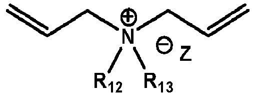 where R₁₂ = H, CH₃ or R₁₃, R₁₃ = any linear or branched carbon chain containing more than 5 carbon atoms and Z = any Group VII-A halide anion.

### Zwitterionic:

- Ammonioalkanecarboxylates:e.g.2-[(11-(N-ethylacrylamidyl)undecyl)dimethylammonio]acetate 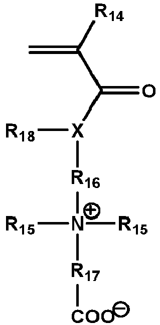 where R₁₄ = H or CH₃, X = O or N, R₁₅ = H, CH₃, CH₂CH₃ or CH₂CH₂OH, R₁₆ = any linear or branched carbon chain more than 5 carbon atoms, R₁₇ = any linear or branched carbon chain containing 5 or less carbon atoms, and R₁₈ = H, CH₃, or nothing.
- Ammonioalkanesulfonates: e.g. 3 [(11methacryloyloxyundecyl)dimethylammonio]propanesulfonate 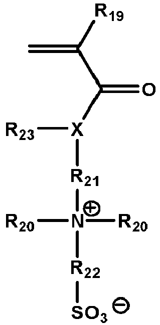 where R₁₉ = H or CH₃, X = O or N, R₂₀ = H, CH₃, CH₂CH₃ or CH₂CH₂OH, R₂₁ = any linear or branched carbon chain more than 5 carbon atoms, R₂₂ = any linear or branched carbon chain containing 5 or less carbon atoms, and R₂₃ = H, CH₃, or nothing.

### Nonionic:

- ω-methoxypoly(ethyleneoxy)alkyl- α -(meth)acrylates: e.g. ω-ethoxypoly(ethyleneoxy)undecyl- α -methacrylate 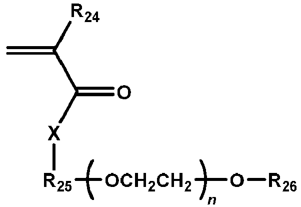 where R₂₄ = H or CH₃, X = O, R₂₅ = any linear or branched carbon chain more than 5 carbon atoms, n is an integer from about 4 to about 800, and R₂₆ = any linear or branched carbon chain containing 5 or less carbon atoms
- ω-alkoxypoly(ethyleneoxy)-α-(meth)acrylates and ω-alkoxypoly(ethyleneoxy)-α-itaconates: e.g. steareth-20 methacrylate, ceteth-20 itaconate 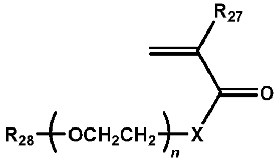 where R₂₇ = H, CH₃, or CH₂COOH, X = O, R₂₈ = any linear or branched carbon chain more than 5 carbon atoms, and *n* is an integer from about 4 to about 800

Nonlimiting examples of ethylenically unsaturated superhydrophilic comonomers include the following, and the like:
Nonionic:
   - glyceryl (meth)acrylate
   - sucrose mono(meth)acrylate, glucose
      mono(meth)acrylatetris(hydroxymethyl)acrylamidomethane, 1-(2-(3-(allyloxy)-2-hydroxypropylamino)ethyl)imidazolidin-2-one (Sipomer^{®} WAM from Rhodia)
Anionic:
   - itaconic acid, hydrophilic derivatives thereof, and alkali metal salts thereof
   - crotonic acid, hydrophilic derivatives thereof, and alkali metal salts thereof
   - maleic acid, hydrophilic derivatives thereof, and alkali metal salts thereof
Cationic:
   - 2-(meth)acryoyloxy-N-(2-hydroxyethyl)-N,N-dimethylethylammonium chloride, 3-(meth)acrylamido-N-(2-hydroxyethyl)-N,N-dimethylpropylammonium chloride, 3-(meth)acrylamido-N,N-bis(2-hydroxyethyl)-N-methylpropylamonium chloride, N-(2-(bis(2-hydroxyethyl)amino)ethyl)(meth)acrylate, N-(3-(bis(2-hydroxyethyl)amino)propyl)(meth)acrylamide, N-(2-((meth)acryloyloxy)ethyl)-N,N,N',N',N'-pentamethylethane-1,2-diammonium dichloride
Zwitterionic:
   - 3-[(3-(meth)acrylamidopropyl)dimethylammonio]propanesulfonate, 3-(3-(meth)acrylamidopropyldimethylammonio)propionate, 3-(3-(meth)acrylamidopropyldimethylammonio)acetate, 2-(meth)acryloyloxyethylphosphorylcholine, and the like

Nonlimiting examples of optional ethylenically unsaturated hydrophilic comonomers include the following, and the like:
Nonionic:
   - e.g. acrylamide, N,N-dimethylacrylamide, N-vinylformamide,hydroxyethyl(meth)acrylate, (meth)acrylamidoethylethyleneurea, □-methoxypoly(ethyleneoxy)-□-(meth)acrylate, and the like
Anionic:
   - acrylic acid, β-carboxyethyl acrylate, 2-acrylamido-2-methylpropanesulfonic acid, 3-acrylamido-3-methylbutanoic acid, sodium allylhydroxypropylsulfonate
Cationic:
   - N,N-dimethylaminoethyl methacrylate, N,N-dimethylpropyl (meth)acrylamide, (3-(meth)acrylamidopropyl)trimethylammonium chloride, diallyldimethylammoniumchloride

By way of non-limiting example, SACs made via copolymerization of ethylenically-unsaturated monomers include: 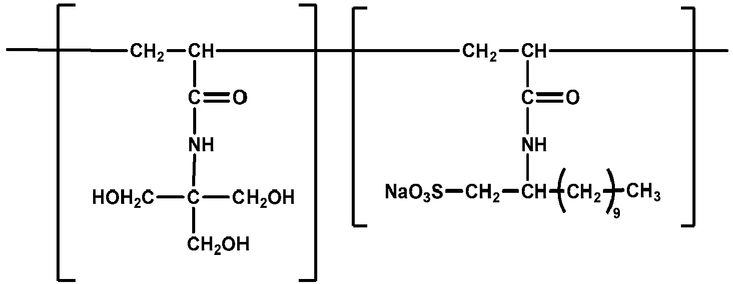 poly[tris(hydroxymethyl)acrylamidomethane-*co*-sodium 2-acrylamidododecylsulfonate] 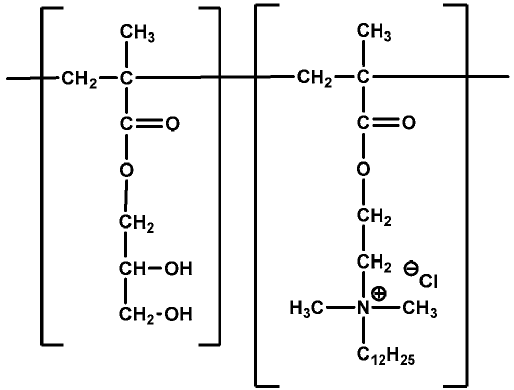 poly[glyceryl methacrylate-*co*-(2-methacryloyloxyethyl)dodecyldimethylammonium chloride]; and the like.

Additional synthetic routes for achieving the SACs of the present invention include via post-polymerization modification of precursor polymers comprising SRUs to render some repeat units amphiphilic. Nonlimiting examples include the reaction of superhydrophilic polymers comprised of repeat units comprising multiple hydroxyl functionalities, for example, starch, hydroxyethylcellulose, dextran, inulin, pullulan, poly(glyceryl methacrylate), poly[tris(hydroxymethyl)acrylamidomethane)], or poly(sucrose methacrylate), with reagents that will result in amphiphilic repeat units.

Examples of suitable reaction schemes include:
i) Esterification with alkenyl succinic anhydrides
ii) Etherification with 1,2-epoxyalkanes
iii) Etherification of with 3-chloro-2-hydroxypropylalkyldimethylammonium chlorides
iv) Esterification with monoalkyl phosphate esters

According to certain embodiments, the SAC for use in the present invention is a polymer having multiple hydroxyl functionalities that is then post-polymerization modified to convert some of the repeat units to ARUs. In one embodiment, the polymer, e.g., a starch such as a starch dextrin polymer, that is esterified with an alkenyl succinic anhydride to convert some of the superhydrophilic anhyroglucose units to ARUs. The structure of one such suitable resulting SAC may be the C-6 sodium dextrin alkenylsuccinate, represented below:

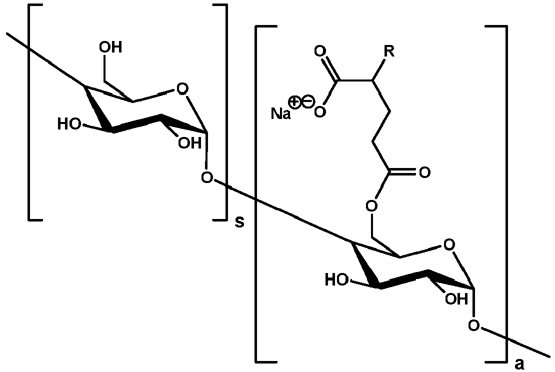

For example, the SAC may be a sodium dextrin dodecenylsuccinate, if R = C₁₂H₂₃. As will be recognized by one of skill in the art, such alkenyl succinate esters of polysaccharides may be synthesized as described, for example, in U.S. 2,661,349, incorporated herein by reference. Depending on the nature of the reaction conditions, molecular architecture, type of sugar repeat units, branch points and molecular weight, the modification of the sugar repeat units (AGU) may also occur at the C-2, C-3 or C-4 positions in addition to the C-6 position shown above.

The SACs derived from the reaction of the starting polysaccharide with the hydrophobic reagent comprises a polysaccharide bound with the hydrophobic reagent. In certain embodiments, the SAC is a starch based polysaccharide modified with one or more hydrophobic reagents. Examples of suitable starches include those derived from such plants as corn, wheat, rice, tapioca, potato, sago, and the like. Such starches can be of a native variety or those developed by plant breeding or by gene manipulation.

In an embodiment of the invention, the starches include either the waxy versions of such starches (containing less than 5% by weight amylose), high amylose starches (containing more than 40% by weight amylose), those with a modified chain length (such as those disclosed in U.S. Patent No. 5,954,883, which is incorporated by reference in its entirety herein), and/or combinations thereof. In certain embodiments, the starting starch is potato starch or tapioca starch. In certain other embodiments, the starting starch is a waxy potato starch or waxy tapioca starch.

In certain embodiments, the starch-based polysaccharide is modified by dissolving such low molecular weight starch or "dextrin" in water and reacting such starch with a hydrophobic reagent. The starch is desirably processed to lower its molecular weight by techniques known in the art, e.g., action of acid and heat, enzymatic, or thermal processing. The low molecular weight starch is dissolved in water, with optional heating, to form an aqueous solution and the pH of the aqueous solution is adjusted to about 2 by addition of an acid, such as a mineral acid (e.g. hydrochloric acid), to the solution. To minimize the removal of water at the end of the reaction, it is preferred that the starch solution be prepared at the highest solids possible. In an exemplary embodiment, a suitable working range for aqueous solids of the low molecular weight starch is from about 10% to about 80% by weight starch based on the total weight of the solution. Preferably, the percent solids of the low molecular weight starch is from about 25% to about 75% by weight based on total weight of solution. In another embodiment, the percent solids of the low molecular weight starch may be from about 35% to about 70% by weight of the total solution.

The viscosity of an aqueous solution of the SAC is desirably low to minimize the detrimental effect of a high solids level of surfactant with pumping or flow of the solution. For this reason, in an embodiment of the invention, the Brookfield viscosity measured at room temperature (about 23°C) at 200 rpm using spindle #3 for the SACs of this invention may be less than about 1000 cps at 10% aqueous solids based on the total weight of the solution. In another embodiment, the Brookfield viscosity measured at room temperature (about 23°C) at 200 rpm using spindle #3 of the 10% aqueous solution may be less than about 25 cps. In yet another embodiment, the Brookfield viscosity measured at room temperature (about 23°C) at 200 rpm using spindle #3 of a 10% aqueous solution will be less than about 10 cps. In a further step, the conversion of some of the superhydrophilic anhydroglucose units to ARUs is performed by reacting one or more hydrophobic reagents (e.g., alkenyl succinic anhydride) with the starch in the aqueous solution at a pH of about 8.5 at about 40_{°}C for about 21 hours to form an aqueous solution of SAC. Additional process steps such as cooling the aqueous solution of SAC to about 23°C and neutralizing the solution to a pH of about 7 may then be performed. In an embodiment of the invention, the pH is adjusted by using a mineral acid, such as hydrochloric acid.

In certain embodiments, the starch-based polysaccharide is modified with alkenyl succinic anhydride. In certain embodiments, the alkenyl succinic anhydride is dodeceneylsuccinic anhydride (DDSA). Exemplary treatment levels of the DDSA, on the dry basis of low molecular weight ranges from about 3% to about 25% by weight. In another embodiment, the treatment level may be from about 5% to about 15% DDSA by weight, based on the dry weight of low molecular weight starting starch.

In an embodiment of the invention, the SACs derived from the reaction of the starting polysaccharide and DDSA, the bound DDSA on the starch-based polysaccharide may be from about 3% about 15% by weight, based on the weight of dry starch. In another embodiment, the bound DDSA will be between 5% and 12% by weight, based on the dry weight of starch.

In an embodiment of the invention, the solution containing the low molecular weight polysaccharide may then be contacted with the DDSA using sufficient agitation to keep the DDSA uniformly dispersed throughout the solution. The reaction may then be run at temperatures between 25°C and 60°C while the pH of the reaction is kept from about 7 and about 9 by the slow and controlled addition of a suitable base. Some examples of such suitable base materials include, but not limited to, sodium hydroxide, potassium hydroxide, sodium, carbonate, potassium carbonate and calcium oxide (lime) and the like.

In an exemplary embodiment of the invention, the hydrophobic reagent is a highly branched version of DDSA containing a 12 carbon side chain made from tetramerization of propene. It has been found that when the tetrapropene is then reacted with maleic anhydride in an ene-type reaction, it forms highly branched tetrapropenyl succinic anhydride (TPSA). Because this material is a slightly viscose oil and has acceptable water solubility (e.g., at about 2-5% in water at 23°C), this reagent is capable of reacting favorably with the low molecular weight polysaccharide. In an embodiment of this invention, therefore, the hydrophobic reagent used to modify the low molecular weight starch may be TPSA.

In certain other embodiments, the starch-based polysaccharide is modified with a long chain quaternary compound having at least one chain containing 3 or more carbon atoms. In another embodiment the long chain quaternary compound has at least one chain containing 6 or more and more preferably 12 or more carbon atoms, such as 3-chloro-2-hydroxpropyl-dimethyldodecylammonium chloride (sold commercially as QUAB(r) 342) or the epoxide form of such compound, 2,3epoxypropyldimethyldodecylammonium chloride.

In still another embodiment of the invention, the one or more hydrophobic reagents may be a combination of reagents, for example, a succinic anhydride and a long chain quaternary ammonium compound. A dialkylanhydride, such as stearyl anhydride, may also be suitable in the present invention.

In a further embodiment, the hydrophobic reagent has a molecular weight of about 220 or greater. Preferably, the hydrophobic reagent has a molecular weight of about 250 or greater. In a further embodiment, the hydrophobic reagent has a molecular weight about 200,000 or less.

In certain embodiments, the modified starch-based polysaccharide has a weight average molecular weight of below 200,000. In certain embodiments, the modified starch-based polysaccharide has a weight average molecular weight of from about 1,000 to about 25,000 or about 1,500 to about 15,000, or about 3,000 to about 10,000.

In addition to starch-based polysaccharides, other polysaccharides are suitable for use in the present invention. Such polysaccharides may be derived from plant sources and those based on sugar-type repeat units. Some non-limiting examples of these polysaccharides are guar, xanthan, pectin, carrageenan, locust bean gum, and cellulose, including physical and chemically modified derivatives of the above. In embodiments of the invention, physical, chemical and enzymatic degradation of these materials may be necessary to reduce the molecular weight to the desired range to provide the viscosity for the desired application. Chemical modification can also be performed to provide additional functional properties (e.g., cationic, anionic or non-ionic) such as treatment with propylene oxide (PO), ethylene oxide (EO), alkyl chlorides (alkylation) and esterification such as 3-chloro-2-hydroxypropyltrimethylammonium chloride, sodium tripolyphosphate, chloroacetic acid, epichlorohydrin, phosphorous oxychloride and the like.

Another non-limiting example of a SAC derived from post-polymerization modification of a polysaccharide includes: 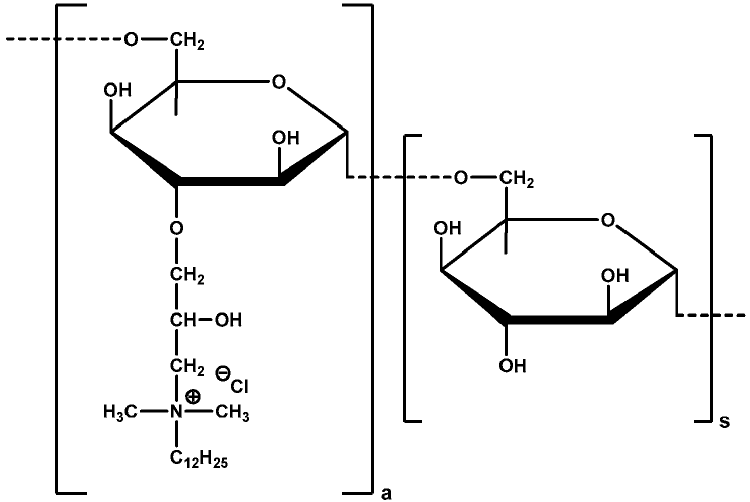 Dextran (poly[α(1→6)-*D*-glucose]) modified with 3-chloro-2-hydroxypropyllauryldimethylammonium chloride; and the like.

Other synthetic routes may include polymerization of amino acids and/or postpolymerization modification of polyaminoacids to achieve a SAC of the present invention, as well as, post-polymerization modification of hydrophilic polymers or amphiphilic polymers to achieve SACs of the present invention, and the like.

According to certain embodiments, the SAC is used in a concentration from about 0.1% to about 25% by weight of active SAC in the composition. Preferably, the SAC is in a concentration from about 0.5% to about 10%, or from about 0.75% to about 6.0% of active SAC in the composition.

### UV-ABSORBING COMPOUND

Embodiments of the invention relate to compositions including an ultraviolet radiation-absorbing compound, (*i.e.,* "UV-absorbing compound"). By "UV-absorbing compound," it is meant a compound comprising one or more UV-absorbing moieties, as discussed herein below, and that absorbs radiation in some portion of the ultraviolet spectrum (290nm-400nm), such as one having an extinction coefficient of at least about 1000 mol⁻¹ cm⁻¹, for example greater than 10,000, or 100,000, or 1,000,000 mol⁻¹ cm⁻¹, for at least one wavelength within the above-defined ultraviolet spectrum.

In certain embodiments of the invention, the UV-absorbing compound may have low water solubility. For example, in certain embodiments, the UV-absorbing compound may have a water solubility that is about 3% or less by weight, such as about 1% or less by weight. By "water solubility" it is meant the maximum weight percentage of UV-absorbing compound (relative to polymer plus water) that can be placed into 100 grams deionized water and agitated so that a clear solution is obtained and remains visually homogeneous and transparent at ambient temperature for 24 hours.

The UV-absorbing compound includes one or more UV-absorbing moieties. In one particular embodiment, the first ultraviolet-absorbing moiety is a UV-A absorbing moiety By "UV-A absorbing moiety," it is meant a moiety that confers appreciable absorbance in the UV-A portion (320nm to 400 nm) of the ultraviolet spectrum to the UV-absorbing compound. For example, when a compound that includes the UV-absorbing polymer is cast into a film, it is possible to generate a molar extinction coefficient measured for at least one wavelength in this wavelength range of at least about 1000 mol⁻¹ cm⁻¹, such as at least about 2000 mol⁻¹ cm⁻¹, such as at least about 4000 mol⁻¹ cm⁻¹. In one embodiment, the molar extinction coefficient among at least 40% of the wavelengths in this portion of the spectrum is at least about 1000 mol⁻¹ cm⁻¹.

Examples of moieties that are UV-A absorbing include tertrahydroxybenzophenones; dicarboxydihydroxybenzophenones and alkane ester or acid halide derivatives thereof; dihydroxy-, dicarboxy-, and hydroxycarboxydibenzoylmethanes and alkane ester or acid halide derivatives thereof; dihydroxy-, dicarboxy-, and hydroxycarboxystilbenes and alkane ester or acid halide derivatives thereof; bis(hydroxystyrenyl) benzenes; bis(carboxystyrenyl)benzenes and alkane ester or acid halide derivatives thereof; dihydroxy-, dicarboxy, and hydroxycarboxycarotenes and alkane ester or acid halide derivatives thereof; 2 cyano - 3,3 - diphenyl acrylic acid, 2-ethyl hexyl ester; and any suitably functionalized species capable of absorbing ultraviolet light in the 320-400 nm range.

In one embodiment, the UV-absorbing moiety is a UV-absorbing triazole and/or a UV-absorbing benzoylmethane. In a particularly notable embodiment, the UV-absorbing moiety is a UV-absorbing triazole.

By "UV-absorbing triazole" it is meant a UV-absorbing moiety containing a five-member heterocyclic ring with two carbon and three nitrogen atoms. UV-absorbing triazoles include, for example, compounds of the formula (II) or (III): 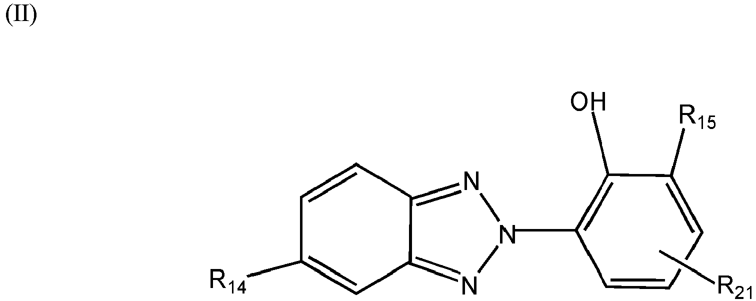 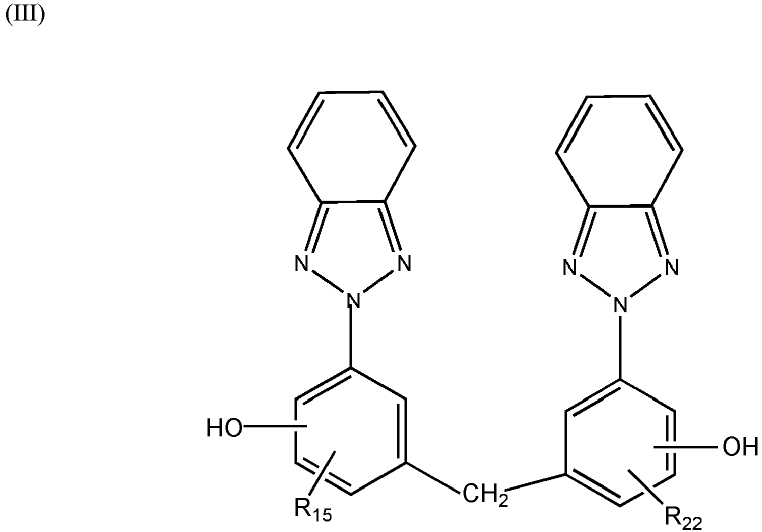 wherein R₁₄ is an optional C₁-C₁₈ alkyl or hydrogen; R₁₅ and R₂₂, independently, are optionally C₁-C₁₈ alkyl that may be substituted with a phenyl group, and R₂₁ is an optional C₁-C₈ alkyl. For (II), either of the R₁₄, R₁₅, or R₂₁ group may be oriented so as to be directly bonded to the (ester) linking group that connects the UV-absorbing dibenzoylmethane to the C-C backbone. For (III), either of the R₁₅ or R₂₂ group may be oriented so as to be directly bonded to the (ester) linking group that connects the UV-absorbing triazole to the C-C backbone.

UV-absorbing dibenzoylmethanes include those that may be represented by formula (IV): 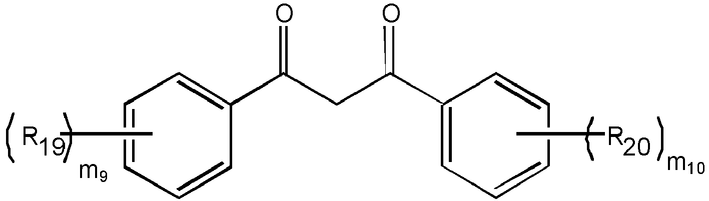 wherein R₁₉ and R₂₀, independently, are optional C₁-C₈ alkyl or C₁-C₈ alkoxy, m₉ is 0 to 3, and m₁₀ is 1 to 3. Either of the R₁₉ and R₂₀ group may be oriented so as to be directly bonded to the (ester) linking group that connects the UV-absorbing dibenzoylmethane to the C-C backbone.

Examples and the synthesis of such dibenzoylmethane moieties are disclosed in U.S. Patent No. 4,489,057 and include, but are not limited to, 4-(1,1-dimethylethyl)-4'-methoxydibenzoylmethane (avobenzone and sold as PARSOL 1789, Roche Vitamins and Fine Chemicals, Nutley, New Jersey, USA).

In another embodiment, the ultraviolet-absorbing moiety is a UV-B absorbing moiety. By "UV-B absorbing moiety," it is meant a moiety that confers appreciable absorbance in the UV-B portion (290nm to 320 nm) of the ultraviolet spectrum. In one embodiment, the criteria for consideration as a UV-B absorbing moiety is similar to those described above for an UV-A absorbing moiety, except that the wavelength range is 290nm to 320 nm.

Examples of suitable UV-B absorbing moieties include 4-aminobenzoic acid and alkane esters thereof; anthranilic acid and alkane esters thereof; salicylic acid and alkane esters thereof; hydroxycinnamic acid alkane esters thereof; dihydroxy-, dicarboxy-, and hydroxycarboxybenzophenones and alkane ester or acid halide derivatives thereof; dihydroxy-, dicarboxy-, and hydroxycarboxychalcones and alkane ester or acid halide derivatives thereof; dihydroxy-, dicarboxy-, and hydroxycarboxycoumarins and alkane ester or acid halide derivatives thereof; and other suitably functionalized species capable of absorbing ultraviolet light in the 290-320 nm range.

In certain embodiment of the invention, "UV-absorbing compound" may be an "organic" UV-absorber. Examples of such compounds, sometimes referred to as "monomeric, organic UV-absorbers" include, but are not limited to, methoxycinnamate derivatives such as octyl methoxycinnamate and isoamyl methoxycinnamate; camphor derivatives such as 4-methyl benzylidene camphor, camphor benzalkonium methosulfate, and terephthalylidene dicamphor sulfonic acid; salicylate derivatives such as octyl salicylate, trolamine salicylate, and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; octyl triazone; butyl methoxydibenzoyl methane; drometrizole trisiloxane; menthyl anthranilate; diethylamino hydroxybenzoyl hexyl benzoate; and bis-ethylhexyloxyphenol methoxyphenyl triazine.

In certain embodiments of the invention, the combinations of UV absorbing compounds may be used, such as but not limited to avobenzone, octocrylene, homosalate, octyl salicylate, and oxybenzone. In other embodiments, combinations of UV absorbing compounds may include avobenzone, octocrylene, homosalate and octyl salicylate. In other embodiments, the UV absorbing compounds may include avobenzone, octocrylene, homosalate, octyl salicylate, and bis-ethylhexyloxyphenol methoxyhenyl triazine.

In yet another embodiment of the invention, UV-absorbing compounds may include UV- absorbing particles typically used at least in part to scatter ultraviolet radiation. Examples include inorganic oxides, including titanium dioxide, zinc oxide, iron oxides, silicone oxides, or other metal (e.g., transition metal, such as crystalline transition metal) oxides. Such ultraviolet screening particles are typically solid particles having a diameter from about 0.1 micron to about 10 microns.

It is further desirable that the UV-absorbing compound have an absorbance in the UV spectrum that is sufficiently high so as to make it suitable for use as a sunscreen for the human body. In one embodiment, the compound, when dissolved in a suitable solvent (e.g., DMSO, ethyl acetate, tetrahydrofuran, or the like) and spread or cast into a thin film, has a molar extinction coefficient measured for at least one wavelength within the UV spectrum, such as in the UV-A spectrum, of at least about 1000 mol⁻¹ cm⁻¹, such as at least about 2000 mol⁻¹ cm⁻¹, such as at least about 4000 mol⁻¹ cm⁻¹, or even 10,000 or 100,000 or 1,000,000 mol⁻¹ cm⁻¹.

A suitable in-vivo test method is the "Colipa Method," known to those skilled in the art. In this method, the minimum dose of solar-simulated ultraviolet radiation (UVR) required to induce a minimally perceptible erythema on human skin is determined for untreated skin and for the skin treated with the composition (erythema readings taken 24 hours after irradiation). The ratio of the dose of UV radiation needed to induce minimally perceptible erythema for the composition-protected skin (MEDp), divided by the dose required for a minimally perceptible erythema for unprotected skin (MEDu) results in the SPF value of the composition.

An irradiation apparatus used for SPF determinations is, for example, a Multiport Solar Simulator Model 601 (Solar Light Co., Philadelphia, Pennsylvania, USA) which consists of a 300 W Xenon lamp filtered with a UG11 1mm thick filter and a WG320 1mm filter (Schott Co., Philadelphia, Pennsylvania, USA) to allow exposure to UV between 240 and 800 nanometers.

### NON-UV-ABSORBING, LIGHT-SCATTERING PARTICLES

Certain embodiments of the invention may include a suspension, or dispersion, of non-ultraviolet (UV) radiation-absorbing, light-scattering particles. By "Non-UV-absorbing light-scattering particles," it is meant particles that do not absorb within the UV spectrum, but may enhance SPF by scattering the incident UV radiation. Such particles significantly boost SPF across the entire UV spectrum across a variety of applications from sunscreens to cosmetics. They perform equally well with both inorganic and organic UV actives and can enhance product feel. Examples of non-UV-absorbing light-scattering particles include solid particles having an average diameter from about 0.1micron to about 10 microns. In certain embodiments, the non-UV-absorbing light-scattering particle is a hollow particle comprising an organic polymer. Suitable organic polymers include acrylic polymers, including acrylic/styrene copolymers, such as those known as SUNSPHERES^{™}PGL, an emulsion, or suspension (25.5% by weight particles in solvent system ), commercially available from Dow Chemical of Midland, Michigan. Such non-UV-absorbing light-scattering particles are suspended in a solvent system, thereby forming a suspension of the particles in the solvent system. Examples of such solvent system include, but are not limited to, water, glycol and mixtures thereof. According to certain embodiments, the solvent system is mixture of water and glycol.

According to certain embodiments, the suspension of the non-UV-absorbing light-scattering particles is added to the composition such that the concentration of the particles in the composition is from about 1% to about 10% by weight. Preferably, the non-UV-absorbing light-scattering particle is in a concentration from about 1% to about 8%, more preferably from about 1% to about 5% by weight of the composition.

### TOPICAL COMPOSITION

The compositions useful in the present invention may be used for a variety of cosmetic uses, especially for protection of the skin from UV radiation. The composition may be employed for various end-uses, such as recreation or daily-use sunscreens, moisturizers, cosmetics/make-up, cleansers/toners, anti-aging products, or combinations thereof. The compositions of the present invention may be prepared using methodology that is well known by an artisan of ordinary skill in the field of cosmetics formulation.

The compositions are in the form of an oil-in-water emulsion containing a continuous water phase and a discontinuous oil phase dispersed within the continuous water phase. In certain embodiments, the oil-in water emulsion is phase stable. As used herein, "phase-stable" means that the water and oil phases of the oil-in-water emulsion do not appreciably separate at room temperature for at least two weeks. In certain embodiments, the UV-absorbing compound is dissolved, as opposed to being dispersed or suspended, within the oil phase. The oil phase may be such that it is present in discrete droplets or units having an average diameter of about one micron to about 1000 microns, such as from about 1 micron to about 100 microns.

According to the present invention, the water phase contains from about 0.75% to about 6% by weight of superhydrophilic amphiphilic copolymers and from about 1.0% to about 5% by weight of the non-UV-absorbing light-scattering particles, based on total weight of the composition.

The percentage by weight of water phase included in the compositions may range from about 45% to about 90%, such as from about 55% to about 80%, such as from about 60% to about 80%. The percentage by weight of water in the water phase may be about 90% or more, such as about 95% or more. In certain embodiments the percentage by weight of oil phase in the composition is from about 10% to about 55%, such as from about 20% to about 45%, such as from about 20% to about 40%.

According to certain embodiments of the present invention, the oil phase consists essentially of a UV absorbing compound and the oil phase is substantially free of oil soluble polymers. By "substantially free of an oil soluble polymer", it is meant that the oil phase does not contain an oil soluble polymer at levels that will detrimentally affect the quality of foam generated by the foaming composition, or prevent the foaming composition from forming such foam when applied; for example, about 3% by weight or less, or about 1% by weight or less, or about 0.75% by weight or less. By "Oil soluble polymer" it is meant any polymeric material that is soluble in the oil phase of the composition. Examples of oil soluble polymers include, but are not limited to, a polyamide, e.g. Sylvaclear WF1500V (Polyamide-4), available from Arizona Chemical, an emollient, e.g. siliconyl carnauba wax, commercially available from Koster Keunen Inc., or oil, e.g., a film-forming polymer such as GANEX V216, commercially available from Ashland Specialty Ingredients of Wayne, NJ, and a non-ionic emulsifer such as Myrj-S100, available from Croda Inc. In certain embodiment, the oil phase consists essentially of the UV absorbing compound. In certain embodiments, the oil phase contains about 80% or more by weight of the UV absorbing compound, such as greater than 90% by weight of the UV absorbing compound, such as greater than 95% by weight of the UV absorbing compound, such as greater than 97% by weight of the UV absorbing compound, such as greater than 99% by weight of the UV absorbing compound.

In contrast, water soluble or water dispersible polymers may be added to the compositions. The water dispersible polymers are comprised of a water-insoluble polymer that is typically micronized and dispersed into a water carrier, possibly with the use of a surface active dispersing aid. The water dispersible polymeric film formers are capable of forming a film and improving water resistance of the compositions. Examples of water soluble polymers include Polyaldo^{™} 10-1-L (Polyglyceryl-10 Laurate), available from Lonza. Examples of water dispersible polymeric film formers include water dispersible polyurethanes, such as Baycusan^{®} C1000 (Polyurethane-34), available from Bayer, Dow Corning^{®} 2501 (Bis-PEG-18 Methyl Ether Dimethyl Silane), available from Dow Corning, and Eastman AQ^{™} 38S (Polyester-5), available from Eastman Chemical.

In one embodiment, a composition suitable for topical/cosmetic use for application to the human body (e.g., keratinaceous surfaces such as the skin or hair), especially the skin, is provided. The composition includes one or more UV-absorbing compound described herein. In certain embodiments the concentration by weight of UV-absorbing compound in the composition is about 10% to about 50% by weight, such as from about 15% to about 40% by weight, such as from about 15% to about 35% by weight, such as from about 20% to about 30% by weight.

In certain embodiments the composition may include one or more compounds suitable for enhancing photostability. Photostabilizers include, for example, diester or polyesters of a naphthalene dicarboxylic acid.

### CARRIER

The composition may be combined with a "cosmetically-acceptable topical carrier," i.e., a carrier for topical use that is capable of having the other ingredients dispersed or dissolved therein, and possessing acceptable properties rendering it safe to use topically. As such, the composition may further include any of various functional ingredients known in the field of cosmetic chemistry, for example, emollients, provided that the emollient is not an oil soluble polymer, or the oil phase is substantially free of the emollient where it is oil soluble, as well as other ingredients commonly used in personal care compositions such as humectants, e.g., glycerin, thickeners, opacifiers, fragrances, dyes, solvents for the UV-absorbing compound, among other functional ingredients. In order to provide pleasant aesthetics, in certain embodiments of the invention, the composition is substantially free of volatile solvents, and, in particular C₁-C₄ alcohols such as ethanol and isopropanol.

Furthermore, the composition may be essentially free of ingredients that would render the composition unsuitable for topical use. As such, the composition may be essentially free of solvents such as volatile solvents, and, in particular, free of volatile organic solvents such as ketones, xylene, toluene, and the like. Further the composition may be essentially free of, or free of, propellant gas, such as hydrocarbon gases, including dimethyl ether.

### OIL-IN-WATER EMULSIFIER

Compositions of the present invention include one or more oil-in-water (O/W) emulsifiers selected from a group consisting of anionic emulsifiers and non-ionic emulsifiers. By "emulsifier," it is meant any of a variety of molecules that are suitable for emulsifying discrete oil-phase droplets in a continuous water phase. By "low molecular weigh emulsifiers," it is meant emulsifiers having a molecular weight of about 2000 daltons or less, such as about 1000 daltons or less. The O/W emulsifier may be capable of lowering the surface tension of pure deionized water to 45 dynes per centimeter when added to pure deionized water at a concentration of O/W emulsifier of 0.5% or less by weight at room temperature. The O/W emulsifier may have a hydrophile-lipophile balance (HLB) that is about 8 or more, such as about 10 or more.

Furthermore, the compositions of the present invention are essentially free of, or free of, monomeric surfactant. By "monomeric surfactants" it is meant any surface active agent that is monomeric. The monomeric surfactants may be anionic, nonionic, amphoteric or cationic. Examples of monomeric surfactants include sodium lauryl sulfate or cocoamidopropyl betaine.

In certain embodiments, the composition may include less than 10% by weight, or less than 2% by weight, of an emollient used for the prevention or relief of dryness and for the protection of the skin, as well as solubilizing the UV-absorbing compound. Suitable emollients include mineral oils, petrolatum, vegetable oils (e.g. triglycerides such as caprylic/capric triglyceride), waxes and other mixtures of fatty esters, including but not limited to esters of glycerol (e.g, isopropyl palmitate, isopropylmyristate), and silicone oils such as dimethicone. In certain embodiments, mixtures of triglycerides (e.g. caprylic/capric triclycerides) and esters of glycols (e.g. isopropyl myristate) may be used to solubilize the UV-absorbing compounds. In other embodiments of the invention, the compositions are essentially free of, or free of, emollients.

In certain embodiments, the composition includes a pigment suitable for providing color or hiding power. The pigment may be one suitable for use in a color cosmetic product, including compositions for application to the hair, nails and/or skin, especially the face. Color cosmetic compositions include, but are not limited to, foundations, concealers, primers, blush, mascara, eyeshadow, eyeliner, lipstick, nail polish and tinted moisturizers. The pigment suitable for providing color or hiding power may be composed of iron oxides, including red and yellow iron oxides, titanium dioxide, ultramarine and chromium or chromium hydroxide colors, and mixtures thereof. The pigment may be a lake pigment, e.g., an organic dye such as azo, indigoid, triphenylmethane, anthraquinone, and xanthine dyes that are designated as D&C and FD&C blues, browns, greens, oranges, reds, yellows, etc., precipitated onto inert binders such as insoluble salts. Examples of lake pigments include Red #6, Red #7, Yellow #5 and Blue #1. The pigment may be an interference pigment. Examples of interference pigments include those containing mica substrates, bismuth oxycloride substrates, and silica substrates, for instance mica/bismuth oxychloride/iron oxide pigments commercially available as CHROMALITE pigments (BASF), titanium dioxide and/or iron oxides coated onto mica such as commercially available FLAMENCO pigments (BASF), mica/titanium dioxide/iron oxide pigments including commercially available KTZ pigments (Kobo products), CELLINI pearl pigments (BASF), and borosilicate-containing pigments such as REFLECKS pigments (BASF).

The compositions of the present invention may further comprise one or more other cosmetically active agent(s). A "cosmetically active agent" is a compound that has a cosmetic or therapeutic effect on the skin, e.g., agents to treat wrinkles, acne, or to lighten the skin. The cosmetically active agent will typically be present in the composition of the invention in an amount of from about 0.001% to about 20% by weight of the composition, e.g., about 0.01% to about 10% by weight, such as about 0.1% to about 5% by weight of the composition.

The compositions of the present invention may be prepared using mixing and blending methodology that is well known by an artisan of ordinary skill. In one embodiment of the invention, a method of making a composition of the present invention includes preparing an oil phase by mixing at least the UV-absorbing compound with optional oil soluble or oil-miscible ingredients; and preparing a water phase, by mixing water and optional water-soluble or water-miscible ingredients. The oil phase and the water phase may then be mixed in a manner sufficient to disperse the oil phase substantially homogeneously in the water phase such that the water phase is continuous and the oil phase discontinuous.

The compositions of the present invention can be used by topically administering to a mammal, e.g., by the direct laying on, wiping or spreading of the composition on the skin or hair of a human.

### EXAMPLES

The following examples illustrate the preparation and efficacy of compositions of the present invention.

### Example I EpiOcular^{™} Scores

The following example illustrates the mildness of certain compositions of the present invention. Inventive compositions (E1-E4) include sodium hydrolyzed potato starch dodecenylsuccinate, are free of monomeric surfactant, e.g., cocamidopropyl betaine, and further include Polyglyceryl-10 Laurate. Comparative compositions include monomeric surfactant cocamidopropyl betaine. These examples were prepared as shown in table 1 and described below:
Inventive Examples E1 through E4 were made by the following process: A water phase was prepared by premixing KELTROL CG-F in PROPYLENE GLYCOL to a main vessel then EDTA-NA2, POLYALDO 10-1-L, EUXYL PE9010, and WATER were added to the mixture while heating to 65°-70°C temperature and mixing with propeller mixer. Then, NATRASURF^{™} PS-111 was added and mixed until uniform. An oil phase was prepared by mixing NEOHELIOPAN HMS, NEOHELIOPAN OS/BP, NEOHELIOPAN 357, NEOHELIOPAN BB, NEOHELIOPAN 303, and DL-A-TOCOPHERYL ACETATE while heating to 65°C -70°C temperature. Both phases were emulsified by adding the oil phase to the water phase while homogenizing with Silverson L4RT at 8000-9000 rpm until uniform. The emulsified phases were mixed by switching back to the propeller mixer at low speed and cooled to room temperature. Then, SUNSPHERES PGL dispersion/suspension was added and mixed uniformly to the emulsion. The composition was then transferred to the appropriate pump foam container, as described herein above.

Comparative Examples C1 and C2 were prepared as shown in table 1 by the process as described above.

### Epi-Ocular^{®} Test:

The potential for irritation to the eyes expected for a given formulation is measured in accordance with the "Epi-Ocular^{®} Test" as set forth below. The Epi-Ocular Test is a cell based in-vitro assay in which cell viability is assessed by measuring the activity of cell enzymes that can reduce MTT (3-(4,5-Dimethylthiazol-2-yl)-2,5-diphenyltetrazolium bromide). The exposure time required for a 50% decrease in the viability of the cells in a 3-D scaffold is reported as the EPI-OCULAR VALUE for the composition. An EPI-OCULAR VALUE>10 hours is considered to be particularly mild, while an EPI-OCULAR VALUE >24 hours is even more desirable.

EpiOcular^{®} OCL-200 tissue (differentiated human epidermal keratinocytes) and assay media are provided by MatTek Corporation of Ashland, Mass. The tissues are stored at 2°-8° C until ready for use. On the day of testing, the tissues are warmed to 37° C in 1 ml of fresh media. Duplicate tissues are dosed topically with 100 microliters of test composition, positive control (0.3% Polyethylene glycol octylphenyl ether, CAS No. 9002-93-1. available from Fisher Scientific Fairlawn, N.J.), or negative control (sterile water). Tissues are incubated for 24 hours, then removed and rinsed with phosphate buffer solution, incubated for ten minutes at room temperature in fresh media, then placed in a 24-well plate containing 0.3 ml of 1 mg/mL of MTT in MTT Addition Medium supplied by MatTek and incubated in the dark for approximately 3 hours. Following incubation with MTT, the medium is decanted and the reduced intracellular MTT is extracted from each tissue construct using 2 ml of isopropanol and orbital shaking at room temperature for 2 hours. Two hundred microliter aliquots of the extract solution are transferred to a 96-well plate and read on a plate reader for optical density at 540-550 nm. Percent Viability for each exposure time point is calculated by dividing mean optical density (OD) of the test material by that of the negative control, where the negative control represents 100% viability, and multiplying the result by 100. Percent Viability is plotted versus time on a semi-log scale and exposure time required for a 50% decrease in cell viability (i.e., ET50, or "EPI-OCULAR VALUE") is extrapolated from the plot. The test is considered valid if 1) the positive control causes an ET50 within two deviations of the historical mean and 2) the mean optical density of the negative control at the shortest and longest time points are within 20%.

Additional details of the test are described in the following publication: McCain, N. E., Binetti, R. R., Gettings, S. D., Jones, B. C. Cell Biology & In Vitro Toxicology, Avon Products, Inc., Suffern, N.Y. The Toxicologist, 66 (1-S), 243, Soc. of Toxicol. (Reston, Va.).

**Table 1 EpiOcular^{™} Scores**

| | | **E1** | **C1** | **E2** | **C2** | **E3** | **E4** |
|---|---|---|---|---|---|---|---|
| **Sunscreen Foam** | | **0.75% PS111** | **Cocamidopropyl betain (0.75% active)** | **3% PS111** | **Cocamidopropyl betain (3% active)** | **0.75% PS111 w/o 10-1-L** | **3% PS111 w/o 10-1-L** |
| | | | | | | | |

| **Trade name** | **Ingredient** | | | | | | |
|---|---|---|---|---|---|---|---|
| NATRASURF^{™} PS-111 | Sodium Hydrolyzed Potato Starch Dodeceny lsuccinate | 0.75 | | 3 | | 0.75 | 3 |
| Neo Heliopan HMS | Homosalate | 10 | 10 | 10 | 10 | 10 | 10 |
| Neo Heliopan OS/BP | Octisalate | 5 | 5 | 5 | 5 | 5 | 5 |
| NEO HELIOPAN 357 | Avobenzone | 3 | 3 | 3 | 3 | 3 | 3 |
| Neo Heliopan BB | Oxybenzone | 6 | 6 | 6 | 6 | 6 | 6 |
| Neo Heliopan 303 | Octocrylene | 4 | 4 | 4 | 4 | 4 | 4 |
| dl-a-TOCOPHERYL ACETATE | Tocopheryl Acetate | 0.2 | 0.2 | 0.2 | 0.2 | 0.2 | 0.2 |
| EDTA-Na2 | Disodium EDTA | 0.1 | 0.1 | 0.1 | 0.1 | 0.1 | 0.1 |
| Propylene Glycol | Propylene Glycol | 1 | 1 | 1 | 1 | 1 | 1 |
| KELTROL CG- F | Xanthan Gum | 0.2 | 0.2 | 0.2 | 0.2 | 0.2 | 0.2 |
| Polyaldo 10-1-L | Polyglyceryl-10 Laurate | 0.25 | | 0.25 | | | |
| Tego Betain F 50 | Cocamidopropyl Betaine, Sodium Chloride, Water | | 1.97 | | 7.9 | | |
| Euxyl PE 9010 | Phenoxyethanol and ethylhexylglycerin | 0.8 | 0.8 | 0.8 | 0.8 | 0.8 | 0.8 |
| Sunspheres PGL Dispersion | Styrene/Acrylates Copolymer | 10 | 10 | 10 | 10 | 10 | 10 |
| DI water | Water | 58.7 | 57.73 | 56.45 | 51.8 | 58.95 | 56.7 |
| **TOTAL** | | 100 | 100 | 100 | 100 | 100 | 100 |
| **EPIOCULAR SCORE** | | 13.9 | 1.9 | 5.4 | <1 | 14.1 | 6.7 |
| **2 Wks Room Temperature Stability** | | Pass | Pass | Pass | Pass | Pass | Pass |
| **Foam** | | Yes | Yes | Yes | Yes | Yes | Yes |

| | | | | | | | |
|---|---|---|---|---|---|---|---|
| • Sunspheres PGL dispersion contains about 25.5% of the non-UV absorbing particulate. | | | | | | | |

As shown in Table 1, formulations containing sodium hydrolyzed potato starch dodecenylsuccinate (E1 to E4) exhibit better EpiOcular^{™} scores compared to formulations containing cocamidopropyl betaine (C1 and C2), indicating compositions free of monomeric surfactant demonstrate enhanced mildness.

### Example II Foam Quality and Foam Performance

The following example illustrates the foam quality and foam performance of certain compositions of the present invention. Inventive compositions (E5-E8) include sodium hydrolyzed potato starch dodecenylsuccinate at a concentration ranging from 0.75% to 6%. Comparative composition (C3) includes sodium hydrolyzed potato starch dodecenylsuccinate at a concentration of 0.375%. These examples were as shown in table 2 and prepared as described above.

**Table 2 Foam Quality and Foam Performance**

| **PS-111 Active Level** | | **E5** | **E6** | **E7** | **E8** | **C3** |
|---|---|---|---|---|---|---|
| **Trade name** | **Ingredient** | | | | | |
| NATRASURF^{™} PS-111 | Sodium Hydrolyzed Potato Starch Dodecenylsuccinate | 6 | 3 | 1.5 | 0.75 | 0.375 |
| Neo Heliopan HMS | Homosalate | 8 | 8 | 8 | 8 | 8 |
| Neo Heliopan OS/BP | Octisalate | 5 | 5 | 5 | 5 | 5 |
| NEO HELIOPAN 357 | Avobenzone | 3 | 3 | 3 | 3 | 3 |
| Neo Heliopan BB | Oxybenzone | 4 | 4 | 4 | 4 | 4 |
| Neo Heliopan 303 | Octocrylene | 4 | 4 | 4 | 4 | 4 |
| dl-a-TOCOPHERYL ACETATE | Tocopheryl Acetate | 0.2 | 0.2 | 0.2 | 0.2 | 0.2 |
| EDTA-Na2 | Disodium EDTA | 0.1 | 0.1 | 0.1 | 0.1 | 0.1 |
| Propylene Glycol | Propylene Glycol | 1 | 1 | 1 | 1 | 1 |
| KELTROL CG- F | Xanthan Gum | 0.2 | 0.2 | 0.2 | 0.2 | 0.2 |
| Polyaldo 10-1-L | Polyglyceryl-10 Laurate | 1.5 | 1 | 0.5 | 0.25 | 0.125 |
| Euxyl PE 9010 | Phenoxyethanol and ethylhexylglycerin | 0.8 | 0.8 | 0.8 | 0.8 | 0.8 |
| DI water | Water | 66.2 | 69.7 | 71.7 | 72.7 | 73.2 |
| **TOTAL** | | 100 | 100 | 100 | 100 | 100 |
| **Foaming** | | Foam | Foam | Foam | Foam | Weak |

As shown in Table 2, formulations containing sodium hydrolyzed potato starch dodecenylsuccinate at a concentration ranging from 0.75% to 6% (E5 to E8) exhibit better foaming compared to formulations containing sodium hydrolyzed potato starch dodecenylsuccinate at a concentration of 0.375% (C3), which exhibited much weaker foaming.

### Example III Foaming Quality and Formulation Phase Stability and Homogeneity

The following example illustrates the phase stability and homogeneity of certain compositions of the present invention. Inventive compositions (E9 -E11) include a suspension of styrene/acrylates copolymer (i.e. SUNSPHERES^{™} PGL). Comparative compositions (C4-C6) include powdered styrene/acrylates copolymer (i.e. SUNSPHERES^{™} powders). These examples are as shown in table 3 and prepared as described above.

The phase stability and homogeneity was determined by visually observing any type of emulsion instability or phase separation such as oil sedimentation, creaming, flocculation, etc. Stability sample at room temperature condition was monitored for 2 weeks duration.

**Table 3 Formulation Phase Stability and Homogeneity**

| **Sunscreen Foam** | | **Active SUNSPHERES^{™} PGL dispersion (25.5% Active)** | | | | **SUNSPHERES^{™} Powders** | | |
|---|---|---|---|---|---|---|---|---|
| | | **E8** | **E9** | **E10** | **E11** | **C4** | **C5** | **C6** |
| **Trade name** | **Ingredient** | | | | | | | |
| NATRASURF^{™} PS-111 | Sodium Hydrolyzed Potato Starch Dodecenylsuccinate | 0.75 | 0.75 | 0.75 | 0.75 | 0.75 | 0.75 | 0.75 |
| Neo Heliopan HMS | Homosalate | 8 | 8 | 8 | 8 | 8 | 8 | 8 |
| Neo Heliopan OS/BP | Octisalate | 5 | 5 | 5 | 5 | 5 | 5 | 5 |
| NEO HELIOPAN 357 | Avobenzone | 3 | 3 | 3 | 3 | 3 | 3 | 3 |
| Neo Heliopan BB | Oxybenzone | 4 | 4 | 4 | 4 | 4 | 4 | 4 |
| Neo Heliopan 303 | Octocrylene | 4 | 4 | 4 | 4 | 4 | 4 | 4 |
| dl-a-TOCOPHERYL ACETATE | Tocopheryl Acetate | 0.2 | 0.2 | 0.2 | 0.2 | 0.2 | 0.2 | 0.2 |
| EDTA-Na2 | Disodium EDTA | 0.1 | 0.1 | 0.1 | 0.1 | 0.1 | 0.1 | 0.1 |
| Propylene Glycol | Propylene Glycol | 1 | 1 | 1 | 1 | 1 | 1 | 1 |
| KELTROL CG- F | Xanthan Gum | 0.2 | 0.2 | 0.2 | 0.2 | 0.2 | 0.2 | 0.2 |
| Polyaldo 10-1-L | Polyglyceryl-10 Laurate | 0.25 | 0.25 | 0.25 | 0.25 | 0.25 | 0.25 | 0.25 |
| Euxyl PE 9010 | Phenoxyethanol and ethylhexylglycerin | 0.8 | 0.8 | 0.8 | 0.8 | 0.8 | 0.8 | 0.8 |
| SUNSPHERES^{™} Powder | Styrene/Acrylates Copolymer | | | | | 1.28 | 2.55 | 3.8 |
| SUNSPHERES^{™} PGL Dispersion | Styrene/Acrylates Copolymer | | 5.0 | 10 | 15 | | | |
| DI water | Water | 72.7 | 67.7 | 62.7 | 57.7 | 71.42 | 70.15 | 68.9 |
| **TOTAL** | | 100 | 100 | 100 | 100 | 100 | 100 | 100 |
| **Foaming** | | Foam | Foam | Foam | Foam | None | None | None |
| **2 wks Stability** | | No | Yes | Yes | Yes | | | |

As shown in Table 3, formulations containing suspension SUNSPHERES^{™} PGL (E9 to E11) exhibit better foam quality compared to formulations containing powdered SUNSPHERES^{™} (C4 to C6). In contrast, formulation containing the powdered SUNSPHERES^{™} failed to yield acceptable foam. Further, formulations containing suspended SUNSPHERES^{™} PGL (E9 to E11) exhibit better phase stability and homogeneity compared to formulation containing no SUNSPHERES^{™} PGL (E8), although compositions containing no SUNSPHERES, whether suspension or powder form, did provide acceptable foam.

### Example IV Formulation Foaming Quality and Performance

The following example illustrates the foaming of certain compositions of the present invention. Inventive compositions (E8 and E12) contain no oil soluble polymer. In contrast, the comparative compositions (C7-C10) contain oil soluble polymer, such as wax, film former, or polymeric emulsifier. These examples were as shown in table 4 and prepared as described above.

**Table 4 Formulation Foaming Quality and Performance**

| **Sunscreen Foam** | | **E8** | **C7** | **C8** | **E12** | **C9** | **C10** |
|---|---|---|---|---|---|---|---|
| **Trade name** | **Ingredient** | **Test 13** | **Test 16** | **Test 17** | **Test 53** | **Test 65** | **Test 66** |
| NATRASURF^{™} PS-111 | Sodium Hydrolyzed Potato Starch Dodecenylsuccinate | 0.75 | 0.75 | 0.75 | 0.75 | 0.75 | 0.75 |
| Neo Heliopan HMS | Homosalate | 8 | 8 | 8 | 8 | 8 | 8 |
| Neo Heliopan OS/BP | Octisalate | 5 | 5 | 5 | 5 | 5 | 5 |
| NEO HELIOPAN 357 | Avobenzone | 3 | 3 | 3 | 3 | 3 | 3 |
| Neo Heliopan BB | Oxybenzone | 4 | 4 | 4 | 4 | 4 | 4 |
| Neo Heliopan 303 | Octocrylene | 4 | 4 | 4 | 4 | 4 | 4 |
| dl-a-TOCOPHERYL ACETATE | Tocopheryl Acetate | 0.2 | 0.2 | 0.2 | 0.2 | 0.2 | 0.2 |
| EDTA-Na2 | Disodium EDTA | 0.1 | 0.1 | 0.1 | 0.1 | 0.1 | 0.1 |
| Propylene Glycol | Propylene Glycol | 1 | 1 | 1 | 1 | 1 | 1 |
| KELTROL CG- F | Xanthan Gum | 0.2 | 0.2 | 0.2 | 0.2 | 0.2 | 0.2 |
| Polyaldo 10-1-L | Polyglyceryl-10 Laurate | 0.25 | 0.25 | 0.25 | | | |
| Myrj S-100 | PEG-100 Stearate | | | | | 1 | |
| Euxyl PE 9010 | Phenoxyethanol and ethylhexylglycerin | 0.8 | 0.8 | 0.8 | 0.8 | 0.8 | 0.8 |
| Sylvaclear WF1500V | Polyamide-4 | | 2 | | | | |
| Siliconyl Carnauba | Carnauba wax | | | 1 | | | |
| SUNSPHERES^{™} PGL Dispersion | Styrene/Acrylates Copolymer | | | | 10 | 10 | 10 |
| Ganex V-216 | VP/Hexadecene Copolymer | | | | | | 3 |
| DI water | Water | 72.7 | 70.7 | 71.7 | 62.95 | 61.95 | 59.95 |
| **TOTAL** | | 100 | 100 | 100 | 100 | 100 | 100 |
| **Foaming** | | Foam | None | None | Foam | Very Weak | None |

As shown in Table 4, formulations containing no oil soluble polymers (E8 and E12) exhibit better foam quality compared to formulations containing oil soluble polymers (C7 to C10). Addition of a polymeric oil soluble wax, film former or emulsifier reduced or killed the foam.

### Example V Formulation Foaming Quality and Performance

The following example illustrates the foaming of certain compositions of the present invention. Inventive composition (E8) contains 99% UV absorbing compound in Oil phase. In contrary, the comparative composition (C11) contains 79% UV absorbing compound in Oil phase. These examples were as shown in table 5 and prepared as described above.

**Table 5. Formulation Foaming as a Function of %UV Absorbing Compound in Oil Phase**

| **SUNSCREEN FOAM - OIL LOAD** | | E8 | C11 |
|---|---|---|---|
| **Trade name** | **Ingredient** | | |
| NATRASURF^{™} PS-111 | Sodium Hydrolyzed Potato Starch Dodecenylsuccinate | 0.75 | 0.75 |
| Finsolv TN | C₁₂-C₁₅ Alkyl Benzoate | | 6 |
| Neo Heliopan HMS | Homosalate | 8 | 8 |
| Neo Heliopan OS/BP | Octisalate | 5 | 5 |
| NEO HELIOPAN 357 | Avobenzone | 3 | 3 |
| Neo Heliopan BB | Oxybenzone | 4 | 4 |
| Neo Heliopan 303 | Octocrylene | 4 | 4 |
| dl-a-TOCOPHERYL ACETATE | Tocopheryl Acetate | 0.2 | 0.2 |
| EDTA-Na2 | Disodium EDTA | 0.1 | 0.1 |
| Propylene Glycol | Propylene Glycol | 1 | 1 |
| KELTROL CG- F | Xanthan Gum | 0.2 | 0.2 |
| Polyaldo 10-1-L | Polyglyceryl-10 Laurate | 0.25 | 0.25 |
| Euxyl PE 9010 | Phenoxyethanol and ethylhexylglycerin | 0.8 | 0.8 |
| DI water | Water | 72.7 | 66.7 |
| **TOTAL** | | 100 | 100 |
| **FOAM** | | Yes | No |
| **%Oil Load** | | 24.2 | 30.2 |

As shown in Table 5, formulations containing no emollients exhibit better foam quality compared to formulations containing amounts of emollient, e.g., Finsolv TN, effective to prevent foaming.

### Example VI Formulation Foaming and Phase Stability

The following example illustrates the phase stability of certain compositions of the present invention. Inventive compositions E13 and E14 contain 28% by weight oil load. In contrast, inventive composition E12 contains 18% by weight oil load. These examples are as shown in table 6 and prepared as described above.

**Table 6. Formulation Foaming and Phase Stability as a Function of Oil Load**

| **Description** | | **E12** | **E13** | **E14** |
|---|---|---|---|---|
| **Trade name** | **Ingredient** | | | |
| NATRASURF^{™} PS-111 | Sodium Hydrolyzed Potato Starch Dodecenylsuccinate | 0.75 | 0.75 | 0.75 |
| Neo Heliopan HMS | Homosalate | | 10 | |
| Tinosorb S | Bis-Ethyhexylophenol Methoxyphenyl Triazine | 2.3 | 2.3 | 3.6 |
| Neo Heliopan OS/BP | Octisalate | 4.5 | 4.5 | 7.0 |
| NEO HELIOPAN 357 | Avobenzone | 3 | 3 | 4.7 |
| Neo Heliopan 303 | Octocrylene | 8 | 8 | 12.4 |
| dl-a-TOCOPHERYL ACETATE | Tocopheryl Acetate | 0.2 | 0.2 | 0.3 |
| EDTA-Na2 | Disodium EDTA | 0.1 | 0.1 | 0.1 |
| Propylene Glycol | Propylene Glycol | 1 | 1 | 1 |
| KELTROL CG- F | Xanthan Gum | 0.2 | 0.2 | 0.2 |
| Euxyl PE 9010 | Phenoxyethanol and ethylhexylglycerin | 0.8 | 0.8 | 0.8 |
| SUNSPHERES^{™} PGL Dispersion | Styrene/Acrylates Copolymer | 10 | 10 | 10 |
| DI water | Water | 69.15 | 59.15 | 59.15 |
| **TOTAL** | | 100 | 100 | 100 |
| **FOAM** | | Yes | Yes | Yes |
| **2 wks stability** | | Slight separation | Stable | Stable |
| **% Oil load** | | **18** | **28** | **28** |

As shown in Table 6, compositions that contain 28% by weight oil load exhibit better phase stability in comparison to compositions that contain 18% by weight oil load, although compositions containing either 18% or 28% by weight oil load, did provide acceptable foam.

### Example VII Foam Quality and Formulation Phase Stability

The following example illustrates the foaming and phase stability of certain compositions of the present invention. Inventive compositions E15 through E17 contain Glycerin, Polyaldo 10-1-L and Dow Corning^{®} 2501 Cosmetic Wax (Bis-PEG-18 Methyl Ether Dimethyl Silane), available from Dow Corning, in addition to SUNSPHERES^{™} PGL. In contrast, inventive composition E13 contains none of these ingredients. These examples were as shown in table 7 and prepared as described above.

The phase stability and homogeneity was determined by visually observing any type of emulsion instability or phase separation such as oil sedimentation, creaming, flocculation, etc.

**Table 7. Formulation Phase Stability as a Function Glycerin, Polyaldo 10-1-L and Dow Corning^{®} 2501Cosmetic Wax**

| **Description** | | **E13** | **E15** | **E16** | **E17** | **E18** |
|---|---|---|---|---|---|---|
| **Trade name** | **Ingredient** | | | | | |
| NATRASURF^{™} PS-111 | Sodium Hydrolyzed Potato Starch Dodecenylsuccinate | 0.75 | 0.75 | 0.75 | 0.75 | 0.75 |
| Tinosorb S | Bis-Ethyhexylophenol Methoxyphenyl Triazine | 2.3 | 2.3 | 2.3 | 2.3 | 2.3 |
| Neo Heliopan OS/BP | Octisalate | 4.5 | 4.5 | 4.5 | 4.5 | 4.5 |
| NEO HELIOPAN 357 | Avobenzone | 3 | 3 | 3 | 3 | 3 |
| Neo Heliopan 303 | Octocrylene | 8 | 8 | 8 | 8 | 8 |
| dl-a-TOCOPHERYL ACETATE | Tocopheryl Acetate | 0.2 | 0.2 | 0.2 | 0.2 | 0.2 |
| EDTA-Na2 | Disodium EDTA | 0.1 | 0.1 | 0.1 | 0.1 | 0.1 |
| Propylene Glycol | Propylene Glycol | 1 | | | | |
| Glycerin | Glycerin | | 5 | 5 | 5 | 5 |
| KELTROL CG- F | Xanthan Gum | 0.2 | 0.2 | 0.2 | 0.2 | 0.2 |
| Polyaldo 10-1-L | Polyglyceryl 10 Laurate | | | 1 | | 1 |
| Euxyl PE 9010 | Phenoxyethanol and ethylhexylglycerin | 0.8 | 0.9 | 0.9 | 0.9 | 0.9 |
| Chlorphenesin | Chlorphenesin | | 0.2 | 0.2 | 0.2 | 0.2 |
| SUNSPHERES^{™} PGL Dispersion | Styrene/Acrylates Copolymer | | 10 | 10 | 10 | 10 |
| Dow Corning 2501 | Bis-PEG-18 Methyl Ether Dimethyl Silane | | | | 1 | 1 |
| DI water | Water | 79.15 | 64.85 | 63.85 | 63.85 | 62.85 |
| **TOTAL** | | 100 | 100 | 100 | 100 | 100 |
| 2 wks RT | | separation two phases | Pass | Pass | Pass | Pass |
| Foam | | OK | OK | OK | OK | OK |

As shown in Table 7, formulations contain Glycerin, Polyaldo 10-1-L and Dow Corning 2501 in addition to SUNSPHERES^{™} PGL (E15 through E17) exhibit improved phase stability compared to formulation contains none of these ingredients (E13), although inventive composition E13 provided an acceptable foam.

### Example VIII Water Soluble/Dispersible Polymer and Foam Quality

The following example illustrates the foam quality of certain compositions of the present invention. Inventive compositions E18 through E20 contain a water soluble/dispersible polymer such as wax, a film former, or a polymeric emulsifier. These examples are as shown in table 8 and were prepared as described above.

**Table 8. Water soluble/dispersible Polymer**

| | | **1% DowCorning^{®} 2501 Cosmetic Wax** | **2% Baycussan C1000 (40% Active)** | **2% Eastman AQ 38S (100% Active)** |
|---|---|---|---|---|
| **Trade name** | **Ingredient** | **E18** | **E19** | **E20** |
| NATRASURF^{™} PS-111 | Sodium Hydrolyzed Potato Starch Dodecenylsuccinate | **0.75** | **0.75** | **0.75** |
| Tinosorb S | Bis-Ethyhexylophenol Methoxyphenyl Triazine | **2.3** | | **2.3** |
| Neo Heliopan HMS | Homosalate | **4.5** | **10** | |
| Neo Heliopan OS/BP | Octisalate | **3** | **5** | **4.5** |
| NEO HELIOPAN 357 | Avobenzone | **8** | **3** | **3** |
| Neo Heliopan BB | Oxybenzone | **0.2** | **6** | |
| Neo Heliopan 303 | Octocrylene | **0.1** | **4** | **8** |
| dl-a-TOCOPHERYL ACETATE | Tocopheryl Acetate | | **0.2** | **0.2** |
| EDTA-Na2 | Disodium EDTA | | **0.1** | **0.1** |
| Propylene Glycol | Propylene Glycol | | **1** | **1** |
| Glycerin | Glycerin | **5** | | |
| KELTROL CG- F | Xanthan Gum | **0.2** | **0.2** | **0.2** |
| Polyaldo 10-1-L | Polyglyceryl-10 Laurate | **1** | **0.25** | |
| Euxyl PE 9010 | Phenoxyethanol and ethylhexylglycerin | **0.9** | **0.8** | **0.8** |
| Chlorphenesin | Chlorphenesin | **0.2** | | |
| Sunspheres PGL Dispersion | Styrene/Acrylates Copolymer | **10** | **10** | **10** |
| DowCorning^{®} 2501 Cosmetic Wax | Bis-PEG-18 Methyl Ether Dimethyl Silane | **1** | | |
| Baycusan C1000 | Polyurethane-34 | | **2** | |
| Eastman Aqua 38S | Polyester-5 | | | **2** |
| DI water | Water | **62.85** | **56.7** | **67.15** |
| **TOTAL** | | **100** | **100** | **100** |
| **Foam** | | **OK** | **OK** | **OK** |

As shown in Table 8, formulations containing water soluble/dispersible polymers (E18 through E20) provided acceptable foam.

### Example IX Viscosity of Formulation

**Table 9. Formulation for Viscosity Measurement**

| | | **E1** |
|---|---|---|
| **Sunscreen Foam** | | **0.75% PS111** |
| **Trade name** | **Ingredient** | |
| NATRASURF^{™} PS-111 | Sodium Hydrolyzed Potato Starch Dodeceny lsuccinate | 0.75 |
| Neo Heliopan HMS | Homosalate | 10 |
| Neo Heliopan OS/BP | Octisalate | 5 |
| NEO HELIOPAN 357 | Avobenzone | 3 |
| Neo Heliopan BB | Oxybenzone | 6 |
| Neo Heliopan 303 | Octocrylene | 4 |
| dl-a-TOCOPHERYL ACETATE | Tocopheryl Acetate | 0.2 |
| EDTA-Na2 | Disodium EDTA | 0.1 |
| Propylene Glycol | Propylene Glycol | 1 |
| KELTROL CG- F | Xanthan Gum | 0.2 |
| Polyaldo 10-1-L | Polyglyceryl-10 Laurate | 0.25 |
| Euxyl PE 9010 | Phenoxyethanol and ethylhexylglycerin | 0.8 |
| Sunspheres PGL Dispersion | Styrene/Acrylates Copolymer | 10 |
| DI water | Water | 58.7 |
| **TOTAL** | | 100 |

### Viscosity of the composition

Material :
   Brookfield Viscometer DV-II + Pro
   RV Spindle 1
   Container : 600 ml Beaker Kimax Kimble No. 14030
Procedure:
   Filled container to 340-350 ml mark
   1 minute reading : 6 rpm, torque 53.3% (to get a consistent reading torque needs to be within 30-70%)
   **Viscosity: 888.3 cps**

As shown in Table 8, formulations containing sodium hydrolyzed potato starch dodecenylsuccinate (E1) exhibits low viscosity.

### Embodiments:

1. A foaming sunscreen composition, comprising:
   an oil-in-water emulsion, comprising
   a continuous water phase comprising from about 0.75% to about 6% by weight of a superhydrophilic amphiphilic copolymer, based on total weight of the foaming sunscreen composition; and
   a discontinuous oil phase dispersed in the water phase,
   wherein the discontinuous oil phase comprises a UV absorbing compound and is substantially free of an oil soluble polymer, and wherein the foaming sunscreen composition is essentially free of monomeric surfactant.
2. The foaming sunscreen composition according to embodiment 1, wherein the superhydrophilic amphiphilic copolymer has a mole percent of amphiphilic unit that is between about 5% and about 10%, and a weight average molecular weight that is about 200,000 or less.
3. The foaming sunscreen composition according to embodiment 1, wherein the oil phase comprises less than 0.1 % by weight of the oil soluble polymers, bases on total weight of the composition.
4. The foaming sunscreen composition according to embodiment 1, wherein the oil phase consists essentially of the UV absorbing compound.
5. The foaming sunscreen composition according to embodiment 1, wherein the oil phase comprises about 80% or more by weight of the UV absorbing compound.
6. The foaming sunscreen composition according to embodiment 1, wherein the oil phase comprises about 95% or more by weight of the UV absorbing compound.
7. The foaming composition of embodiment 1 wherein the composition is essentially free of a propellant gas.
8. The foaming sunscreen composition according to embodiment 1, wherein the UV absorbing compound is selected from the group consisting of aUV-A absorbing moiety and a UV-B absorbing moiety.
9. The foaming sunscreen composition of embodiment 8, wherein the UV-A absorbing moiety is selected from the group consisting of tertrahydroxybenzophenones; dicarboxydihydroxybenzophenones and alkane ester or acid halide derivatives thereof; dihydroxy-, dicarboxy-, and hydroxycarboxydibenzoylmethanes and alkane ester or acid halide derivatives thereof; dihydroxy-, dicarboxy-, and hydroxycarboxystilbenes and alkane ester or acid halide derivatives thereof; bis(hydroxystyrenyl) benzenes, bis(carboxystyrenyl)benzenes and alkane ester or acid halide derivatives thereof; dihydroxy-, dicarboxy, and hydroxycarboxycarotenes and alkane ester or acid halide derivatives thereof; bis-ethylhexyloxyphenol methoxyphenyl triazine; and 2 cyano - 3,3 - diphenyl acrylic acid, 2-ethyl hexyl ester.
10. The foaming sunscreen composition of embodiment 8, wherein the UV-B absorbing moiety is selected from the group consisting of a 4-aminobenzoic acid and alkane esters thereof; anthranilic acid and alkane esters thereof; salicylic acid and alkane esters thereof; hydroxycinnamic acid and alkane esters thereof; dihydroxy-, dicarboxy-, and hydroxycarboxybenzophenones and alkane ester or acid halide derivatives thereof; dihydroxy-, dicarboxy-, and hydroxycarboxychalcones and alkane ester or acid halide derivatives thereof; bis-ethylhexyloxyphenol methoxyphenyl triazine; octyl salicylate; homosalate; oxybenzone; octocrylene; and dihydroxy-, dicarboxy-, and hydroxycarboxycoumarins and alkane ester or acid halide derivatives thereof.
11. The foaming composition of embodiment 1 wherein the UV absorbing compound is selected from the group consisting of octyl salicylate, homosalate, oxybenzone, octocrylene, avobenzone, and Bis-ethyhexylophenol methoxyphenyl triazine).
12. The foaming sunscreen composition according to embodiment 1, wherein a density of the oil phase is at least 0.995g/ml.
13. The foaming sunscreen composition according to embodiment 1, comprising from about 10% to about 55 % by weight of the oil phase.
14. The foaming sunscreen composition according to embodiment 1, comprising from about 20% to 40% by weight of the oil phase.
15. The foaming sunscreen composition according to embodiment 1, comprising from about 10% to about 40% by weight of the UV absorbing compound.
16. The foaming sunscreen composition according to embodiment 1, further comprising a polyol having a density of at least 1g/ml.
17. The foaming sunscreen composition according to embodiment 16, wherein the polyol is glycerin.
18. The foaming sunscreen composition according to embodiment 17, comprising from about 1% to about 20% by weight of the glycerin.
19. The foaming sunscreen composition of embodiment 1, wherein the oil phase comprises about 3% or less by weight of the oil soluble polymer, based on total weight of the foaming sunscreen composition.
20. The foaming sunscreen composition according to embodiment 1 having a viscosity of about 2000 cps or less.
21. The foaming sunscreen composition according to embodiment 1 having a viscosity of about 1000 cps or less.
22. The foaming sunscreen composition according to embodiment 1 further comprising an emulsifier other than the superhydrophilic amphiphilic copolymer.
23. The foaming sunscreen composition of embodiment 22, wherein the emulsifier other than the superhydrophilic amphiphilic copolymer is polyglyceryl 10 laurate.
24. The foaming sunscreen composition of embodiment 1 which is free of an emulsifier other than the superhydrophilic amphiphilic copolymer.
25. The foaming sunscreen composition according to embodiment 1 further comprising a suspension comprising non-UV-absorbing light-scattering particles.
26. The foaming sunscreen composition of embodiment 25 comprising from about 1% to about 10% by weight of the non-UV-absorbing light-scattering particles.
27. The foaming sunscreen composition of embodiment 25 comprising from about 1% to about 5% by weight of the non-UV-absorbing light-scattering particles.
28. The foaming sunscreen composition according to embodiment 27, wherein the non-UV-absorbing light-scattering particles comprise a styrene/acrylate copolymer.
29. The foaming sunscreen composition according to embodiment 25 wherein the oil-in-water emulsion is phase-stable.
30. The foaming composition of embodiment 1 wherein the oil phase comprises about 3% or less by weight of an oil-soluble emollient.
31. The foaming sunscreen composition of embodiment 1 further comprising bis-PEG-18 methyl ether dimethyl silane.
32. The composition of embodiment 1 which is essentially free of an emollient.
33. The foaming sunscreen composition according to embodiment 13, comprising from about 90% to about 45% by weight of the water phase.
34. The foaming sunscreen composition according to embodiment 14, comprising from about 80% to about 60% by weight of the water phase.
35. A non-aerosol mechanical dispensing container comprising a foaming sunscreen composition contained therein, the foaming sunscreen composition comprising:
   an oil-in-water emulsion, comprising
   a continuous water phase comprising from about 0.75% to about 6% by weight of a superhydrophilic amphiphilic copolymer, based on total weight of the foaming sunscreen composition; and
   a discontinuous oil phase dispersed in the water phase,
   wherein the discontinuous oil phase comprises a UV absorbing compound and is substantially free of an oil soluble polymer, the foaming sunscreen composition is essentially free of monomeric surfactant, and the foaming sunscreen composition and the container are free of a propellant gas.

## Claims

1. A foaming sunscreen composition, comprising:
an oil-in-water emulsion, comprising
a continuous water phase comprising from about 0.75% to about 6% by weight of a superhydrophilic amphiphilic copolymer, based on total weight of the foaming sunscreen composition; and
a discontinuous oil phase dispersed in the water phase,
wherein the discontinuous oil phase comprises a UV absorbing compound and is substantially free of an oil soluble polymer, and wherein the foaming sunscreen composition is essentially free of monomeric surfactant.

2. The foaming sunscreen composition according to claim 1, wherein the superhydrophilic amphiphilic copolymer has a mole percent of amphiphilic unit that is between about 5% and about 10%, and a weight average molecular weight that is about 200,000 or less.

3. The foaming sunscreen composition according to claim 1, wherein the oil phase comprises less than 0.1 % by weight of the oil soluble polymers, bases on total weight of the composition.

4. The foaming sunscreen composition according to claim 1, wherein the oil phase:
(i) consists essentially of the UV absorbing compound;
(ii) comprises about 80% or more by weight of the UV absorbing compound;
or (iii) comprises about 95% or more by weight of the UV absorbing compound.

5. The foaming composition of claim 1 wherein the composition is essentially free of a propellant gas.

6. The foaming sunscreen composition according to claim 1, wherein the UV absorbing compound is:
(i) selected from the group consisting of a UV-A absorbing moiety and a UV-B absorbing moiety, optionally wherein:
(a) the UV-A absorbing moiety is selected from the group consisting of tertrahydroxybenzophenones; dicarboxydihydroxybenzophenones and alkane ester or acid halide derivatives thereof; dihydroxy-, dicarboxy-, and hydroxycarboxydibenzoylmethanes and alkane ester or acid halide derivatives thereof; dihydroxy-, dicarboxy-, and hydroxycarboxystilbenes and alkane ester or acid halide derivatives thereof; bis(hydroxystyrenyl) benzenes, bis(carboxystyrenyl)benzenes and alkane ester or acid halide derivatives thereof; dihydroxy-, dicarboxy, and hydroxycarboxycarotenes and alkane ester or acid halide derivatives thereof; bis-ethylhexyloxyphenol methoxyphenyl triazine; and 2 cyano - 3,3 - diphenyl acrylic acid, 2-ethyl hexyl ester;
or (b) the UV-B absorbing moiety is selected from the group consisting of a 4-aminobenzoic acid and alkane esters thereof; anthranilic acid and alkane esters thereof; salicylic acid and alkane esters thereof; hydroxycinnamic acid and alkane esters thereof; dihydroxy-, dicarboxy-, and hydroxycarboxybenzophenones and alkane ester or acid halide derivatives thereof; dihydroxy-, dicarboxy-, and hydroxycarboxychalcones and alkane ester or acid halide derivatives thereof; bis-ethylhexyloxyphenol methoxyphenyl triazine; octyl salicylate; homosalate; oxybenzone; octocrylene; and dihydroxy-, dicarboxy-, and hydroxycarboxycoumarins and alkane ester or acid halide derivatives thereof;
or (ii) selected from the group consisting of octyl salicylate, homosalate, oxybenzone, octocrylene, avobenzone, and Bis-ethyhexylophenol methoxyphenyl triazine.

7. The foaming sunscreen composition according to claim 1, wherein a density of the oil phase is at least 0.995g/ml.

8. The foaming sunscreen composition according to claim 1:
(i) comprising from about 10% to about 55 % by weight of the oil phase, optionally wherein the foaming sunscreen comprises from about 90% to about 45% by weight of the water phase;
(ii) comprising from about 20% to 40% by weight of the oil phase, optionally wherein the foaming sunscreen comprises from about 80% to about 60% by weight of the water phase;
(iii) comprising from about 10% to about 40% by weight of the UV absorbing compound; or
(iv) further comprising a polyol having a density of at least 1g/ml, optionally wherein the polyol is glycerin, further optionally wherein the foaming sunscreen comprises from about 1% to about 20% by weight of the glycerin.

9. The foaming sunscreen composition of claim 1, wherein the oil phase comprises about 3% or less by weight of the oil soluble polymer, based on total weight of the foaming sunscreen composition.

10. The foaming sunscreen composition according to claim 1 having a viscosity of: (i) about 2000 cps or less; or (ii) about 1000 cps or less.

11. The foaming sunscreen composition according to claim 1:
(i) further comprising an emulsifier other than the superhydrophilic amphiphilic copolymer, optionally wherein the emulsifier other than the superhydrophilic amphiphilic copolymer is polyglyceryl 10 laurate;
(ii) which is free of an emulsifier other than the superhydrophilic amphiphilic copolymer; or
(iii) further comprising a suspension comprising non-UV-absorbing light-scattering particles, optionally wherein:
(a) the foaming sunscreen composition comprises: from about 1% to about 10% by weight of the non-UV-absorbing light-scattering particles;
(b) the foaming sunscreen composition comprises from about 1% to about 5% by weight of the non-UV-absorbing light-scattering particles, optionally wherein the non-UV-absorbing light-scattering particles comprise a styrene/acrylate copolymer;
or (c) the oil-in-water emulsion is phase-stable.

12. The foaming composition of claim 1 wherein the oil phase comprises about 3% or less by weight of an oil-soluble emollient.

13. The foaming sunscreen composition of claim 1 further comprising bis-PEG-18 methyl ether dimethyl silane.

14. The composition of claim 1 which is essentially free of an emollient.

15. A non-aerosol mechanical dispensing container comprising a foaming sunscreen composition contained therein, the foaming sunscreen composition comprising:
an oil-in-water emulsion, comprising
a continuous water phase comprising from about 0.75% to about 6% by weight of a superhydrophilic amphiphilic copolymer, based on total weight of the foaming sunscreen composition; and
a discontinuous oil phase dispersed in the water phase,
wherein the discontinuous oil phase comprises a UV absorbing compound and is substantially free of an oil soluble polymer, the foaming sunscreen composition is essentially free of monomeric surfactant, and the foaming sunscreen composition and the container are free of a propellant gas.
